# EUROPEAN PATENT SPECIFICATION

(11) **EP 3 939 544 B1**
(45) Date of publication and mention of the grant of the patent: **18.06.2025**
(21) Application number: 21192166.3
(22) Date of filing: 28.02.2019
(51) Int. Cl.: A61F 2/20, A61B 34/30, A61F 2/48, A61B 17/34

(54) **DYNAMICALLY CONTROLLED SOFT TISSUE MANIPULATOR**
DYNAMISCH GESTEUERTER WEICHGEWEBEMANIPULATOR
MANIPULATEUR DE TISSUS MOUS À COMMANDE DYNAMIQUE

(30) Priority: 09.03.2018 US 201862640964 P
(43) Date of publication of application: 19.01.2022
(62) Divisional of application: 19710939.0
(73) Proprietor: Iotamotion, Inc., Iowa City, Iowa 52240 (US)
(72) Inventor: HOFFMAN, Henry, 165 Kennedy Parkway, Iowa City, Iowa, 52246 (US); KAUFMANN, Christopher, 604 Beldon Ave., Iowa City, Iowa, 52246 (US); REINEKE, Parker, 35 Brunswick Ct., Iowa City, Iowa, 52240 (US); HANSEN, Marlan, 2635 Johnsons Crossing NE, Solon, Iowa, 52333 (US); HAHN, Adam, 10 Midway Rd, Pittsburgh, Pennsylvania, 15216 (US)
(74) Representative: Murgitroyd & Company

(56) References cited:
- WO-A1-2017/048342
- US-A1- 2011 264 038
- US-A1- 2014 276 948

## Description

### CLAIM OF PRIORITY

This application claims the benefit of priority of U.S. Provisional Patent Application Serial Number 62/640,964, filed on March 9, 2018.

### TECHNICAL FIELD

This document relates generally to medical systems and more particularly to systems, devices, and methods for robotic manipulation of an implant to alter position of shape of soft tissue.

### BACKGROUND

Millions of people in the United States and around the world suffer from chronic voice disorders. Many of the chronic voice disorders are associated with vocal cord dysfunction or diseases. Vocal cords are two flexible bands of soft tissue composed of muscle collagen, elastin and ground substance that sit at the entrance to trachea. The two bands are normally positioned apart to allow air to flow during breathing. During speaking, the bands come together to produce sound as the passage of air from the lungs causes them to vibrate to make sound. Appropriate closure of the vocal cords during swallowing and coughing also protects the airway, preventing food, drink, and saliva from entering the trachea.

Vocal cord paralysis (VCP) is a common chronic voice disorder. Vocal cord paralysis is caused by disruption of nerve impulses to the voice box (larynx), resulting in immobility of the vocal cord muscles. VCP can cause hoarseness (dysphonia) most commonly characterized by a breathy or weak voice with roughness. VCP may also cause swallowing problems, and result in chocking leading to death in some extreme cases. In most cases of VCP, only one vocal cord is paralyzed, a condition known as unilateral vocal cord paralysis (UVCP). Dysphonia in patients with UVCP is related to incomplete closure of the vocal cords, such as due to deficient tone and bulk to an improperly positioned paralyzed vocal cord.

Chronic voice disorder may also be age related. Presbylaryngis (aging larynx) generally refers to age-related vocal cord changes including loss of volume and bowing of the vocal cord inner edges. Common symptoms of presbylaryngis may include reduced volume, high pitch, breathy sound, increased speaking effort, vocal fatigue, and difficulty communicating with others. The conformation and volume change in vocal cord edges narrows the gap between the vocal cords during speaking; and other muscles may subsequently push more tightly to compensate for reduced vocal cord closure.

Thyroplasty has been used to treat or alleviate chronic voice disorders associated with conformational change in vocal cords. Thyroplasty is a phono-surgical technique designed to improve patient voice by repositioning an abnormal vocal cord through an opening created in the thyroid cartilage of the voice box. A thyroplasty implant may then be positioned at or near a vocal cord to adjust vocal cord position, bulk and shape. Type I thyroplasty, also known as medialization laryngoplasty, is a surgical procedure that pushes a vocal cord toward the middle of the voice box. It is used for voice disorders resulting from weak or incomplete vocal cord closure, including unilateral vocal cord paralysis, presylaryngis, Parkinson's disease, abductor spasmodic dysphonia, as well as vocal cord atrophy, scar, and paresis (partial paralysis). Type II thyroplasty is a surgical procedure that pulls a vocal cord in lateral direction to weaken vocal cord closure. It has been used to address conditions including adductor spasmodic dysphonia with anticipated application for vocal tremor, refractory muscle tension dysphonia, and bilateral vocal cord paralysis.

WO 2017/048342 A1 describes an implantable device capable of controlling cochlear implant electrode insertion and positioning. The device uses an implanted mechanical positioning unit to advance position and monitor an electrode array. The device can be controlled via an external controller to reposition or advance an electrode array at any point after implantation with no surgical re-intervention.

US 2011/264038 A1 concerns an insertion device allowing a doctor to alone perform an operation to insert a medical linear body. The insertion device, operated to insert a delivery wire into a human body through a blood vessel, includes a foot switch generating and outputting a signal to control starting/stopping a drive device moving the delivery wire in its longitudinal direction.

US 2014/276948 A1 describes a robotic catheter system with a magnetic coupling. The catheter system includes a first drive mechanism comprising a drive which interacts with a catheter device to cause the catheter device to move along its axis. A first encoder assembly that detects the motion of the catheter device by a magnetic interaction with the catheter device.

### SUMMARY

The invention is a system as defined in the appended claims. Methods disclosed herein do not form part of the claimed invention.

Thyroplasty involves surgically implanting an implant at or near a vocal cord in the voice box, and maneuver the vocal cord via the implant to secure the vocal cord into a desired position or to maintain a desired shape. Stabilizing the vocal cord at the appropriate position is critical in managing glottic incompetence (weakened voice production from incomplete vocal cord closure). In a conventional thyroplasty surgery, a surgeon inserts the implant into a patient's voice box by hand. This manual maneuvering of the thyroplasty implant may lack precision in implant positioning and motion control, such as the control of insertion rate, distance, or forces applied to the implant to move the implant to the target site in the voice box. Complete manual maneuvering of the thyroplasty implant may also be subject to high variability among surgeons, which may result in inconsistency in implant positioning. One reason for the inter-operator variability may be related to tissue swelling induced by the implantation surgery. Because of the swelling, it can be difficult for a surgeon to estimate an appropriate amount of medial displacement (e.g., in Type I thyroplasty) or lateral displacement (e.g., in Type II thyroplasty) to be applied to the vocal cords during the surgery. As a result, speculation may be required to account for anticipated post-surgical changes in the position and shape of the vocal cords and surrounding tissue in the ensuing days and weeks as the swelling diminishes. As a result, there can be substantial differences in patient outcomes among institutions and surgeons of differing skill levels. Even experienced thyroplasty surgeons at high-volume institutions have inconsistent results. A recent report from such an institution identified sufficiently poor results at 6 weeks follow-up that 50% of patients were offered revision surgery.

Conventional thyroplasty is subject to high revision rate following the initial surgery. Improvement in vocal quality at the time of surgery may often be followed by deterioration days to weeks later due to resolution of the swelling induced by the surgery, or even years later due to loss of bulk (atrophy) on both the paralyzed cord either due to pressure of the implant or the absence of nerve supply. For these patients, implant revision is often required to reposition the implant to optimize vocal cord position or shape. Because existing thyroplasty implants are static (i.e., lacking capability of flexibility of adjusting implant position or conformation after surgical site closure), a repeat surgery is usually required to modify an existing implant. Repeated surgery not only subjects the patient to additional risk of complication, but also increases complexity and cost for patient management. For these reasons, the conventional thyroplasty procedure is not an optimal long-term solution for many patients with chronic voice disorders.

Less-invasive techniques have been developed to address the repeated intervention associated with thyroplasty implant revision. Injection laryngoplasty is a procedure where a surgeon passes a needle connected to a syringe filled with augmentation material transcutaneously into the vocal cord. The augmentation material is then deposited into the vocal cord to add bulk to one or both of a patient's vocal cords to move its contact area toward the midline, thereby reducing the loss of air and improving the symptoms. Although this approach is less invasive than thyroplasty, gradual resorption of the implant material may occur following the injection, usually in an unpredictable manner. Some studies have shown that injectables made of longer-lasting calcium hydroxyapatite may remain up to 18 months after injection. The resorption may slowly decrease the bulk of the vocal cord, and deteriorates patient voice quality over time. When the resorption occurs, the patient may need repeat injection or alternative longer lasting thyroplasty procedure. For this reason, injection laryngoplasty is considered in many cases to be a temporary solution to correct chronic voice disorders.

For the foregoing reasons, the present inventors have recognized a significant need to improve the medical technology of thyroplasty, particularly to enhance surgical precision in implant delivery and positioning, and flexibility and accuracy in non-invasive revision of an existing thyroplasty implant. The present document discusses, among other things, systems, devices, and methods of robotically assisted positioning of an implant in a patient, and manipulation of the implant to alter position or shape of target soft tissue. The system may include a robotically controlled implantable positioning unit (IPU) that allows a surgeon to remotely and dynamically control the positioning and fine-tune the conformation of the implant. The systems and devices discussed herein may be used not only in an initial implantation surgery, but also in a revision procedure without disruption the skin or adjacent tissue. By way of non-limiting example, the system and devices discussed herein may be used to manipulate a thyroplasty implant, either during initial thyroplasty surgery or subsequent revision procedure, to alter the position, shape, and bulk of a vocal cord to treat various chronic voice disorders, such as medializing a vocal cord to reduce the gap between vocal cords, or lateralizing a vocal cord to weaken vocal cord closure or to enlarge glottis aperture to improve airway opening and ventilation.

The systems and devices discussed herein may improve treatment of many types of voice disorders by enabling non-invasive, transcutaneous control of implant position and conformation to optimize patient vocal quality as age and other factors cause the laryngeal anatomy to evolve over time. In an example, the present system and devices may be used to manage glottic incompetence (incomplete vocal cord closure), such as resulted from aging (presbylaryngis), vocal cord atrophy and scar, or resection of tumors of the vocal cords. In another example, the present system and devices may be used to improve weakened vocal cord closure associated with neurological disorders, such as Parkinsons, abductor spasmodic dysphonia, or vocal tremor. In some examples, the present system and devices may also be used to lateralize the vocal cord to induce or weaken glottic closure in patients with adductor spasmodic dysphonia, refractory muscle tension dysphonia, or vocal tremor.

This summary is intended to provide an overview of subject matter of the present patent application. It is not intended to provide an exclusive or exhaustive explanation of the disclosure. The detailed description is included to provide further information about the present patent application. Other aspects of the disclosure will be apparent to persons skilled in the art upon reading and understanding the following detailed description and viewing the drawings that form a part thereof, each of which are not to be taken in a limiting sense.

### BRIEF DESCRIPTION OF THE DRAWINGS

Various embodiments are illustrated by way of example in the figures of the accompanying drawings. Such embodiments are demonstrative and not intended to be exhaustive or exclusive embodiments of the present subject matter.
FIG. 1 is a block diagram illustrating a robotically assisted and dynamically controlled soft-tissue manipulator system and environrnent in which the soft-tissue manipulator system may operate.
FIGS. 2A-2B illustrate normal vocal cords and those with vocal cord paralysis, a medical condition that may be treated or alleviated by the robotic soft-tissue manipulator system discussed herein.
FIGS. 3A-3C illustrate embodiments of implantable positioning units (IPUs) each coupled to an elongate member of an implant.
FIGS. 4A-4B illustrate embodiments of IPUs for delivering and positioning a guide sheath and an elongate member.
FIGS. 5A-5B illustrate embodiments of IPUs for positioning and manipulating a thyroplasty implant to modify a vocal cord position or shape.
FIGS. 6A-6D illustrate portions of an IPU for positioning and maneuvering a thyroplasty implant and affixation means for affixing the IPU on the thyroid cartilage.
FIGS. 7A-7C illustrate a soft-tissue implant having an array of micro-actuators that can modify position and shape of a target soft tissue.
FIG. 8 is a block diagram illustrating a portion of an external control system to control an IPU to robotically position and manipulate a soft-tissue implant.
FIG. 9 is a flowchart illustrating a method for positioning a soft-tissue implant via a robotically assisted and dynamically controlled tissue manipulator system.
FIG. 10 is a flowchart illustrating a method for robotically controlled positing and manipulation of a soft-tissue implant such as a thyroplasty implant.
FIGS. 11A-11D illustrate different views of an embodiment of an IPU for engaging an elongate member of an implant according to the invention.

### DETAILED DESCRIPTION

In the following detailed description, reference is made to the accompanying drawings which form a part hereof, and in which is shown by way of illustration specific embodiments in which the invention may be practiced. These embodiments are described in sufficient detail to enable those skilled in the art to practice the invention, and it is to be understood that the embodiments may be combined, or that other embodiments may be utilized and that structural, logical and electrical changes may be made without departing from the scope of the present invention. References to "an", "one", or "various" embodiments in this disclosure are not necessarily to the same embodiment, and such references contemplate more than one embodiment. The following detailed description provides examples, and the scope of the present invention is defined by the appended claims.

Disclosed herein are systems, devices, and methods for robotically assisted implantation and manipulation of an implant in a patient to alter a position or a shape of target soft tissue. The present system may be implemented using a combination of hardware and software designed to provide precise control of implant movement, such as insertion of a thyroplasty implant and/or guide sheath during a thyroplasty surgery, or non-invasive revision of an existing thyroplasty implant. An embodiment of the system includes an implantable positioning unit (IPU) configured to engage the implant, deliver and position the implant to interface with the target soft tissue. A user may operate on an external control console to control the IPU to manipulate the implant, and to alter the position and shape of the target soft tissue. In an example, the system may be used in a thyroplasty surgery to position and manipulate a thyroplasty implant to modify a vocal cord, such as to medialize or lateralize the vocal cord to restore or improve voice quality.

Although the discussion in this document focuses on manipulating a thyroplasty implant to alter vocal cords to treat voice disorders, this presentation is meant only by way of example and not limitation. The systems, devices, and methods discussed herein may be used to manage glottic incompetence (incomplete vocal cord closure) resulting from aging (presbylaryngis), vocal cord atrophy and scar, or resection of tumors of the vocal cords; weakened vocal cord closure associated with neurological disorders, such as Parkinsons, adductor spasmodic dysphonia, or vocal tremor; or to induce or weaken vocal cord closure (enhance glottic incompetence) in patients with adductor spasmodic dysphonia, refractory muscle tension dysphonia, or vocal tremor. The systems, devices, and methods discussed herein may additionally be adapted to robotically deliver, steer, position, extract, reposition, or replace various types of implants or prosthesis as well as associated instruments. Examples of the implants may include leads, catheter, guidewire, guide sheath, or other mechanical or electrical devices. The implants may be designed for temporary or permanent implantation. The implants may additionally be used for medical diagnosis of a disease or other conditions such as diagnostic catheters, or for therapeutic purposes of cure, mitigation, treatment, or prevention of disease, such as implantable electrodes for stimulating cardiac, neural, muscular, or other tissues. Through the implant, the system or apparatus may interact with various soft tissue to alter its position, shape, conformation, or contour or topography of a portion thereof to achieve specific diagnostic or therapeutic effects (e.g. tissue expansion).

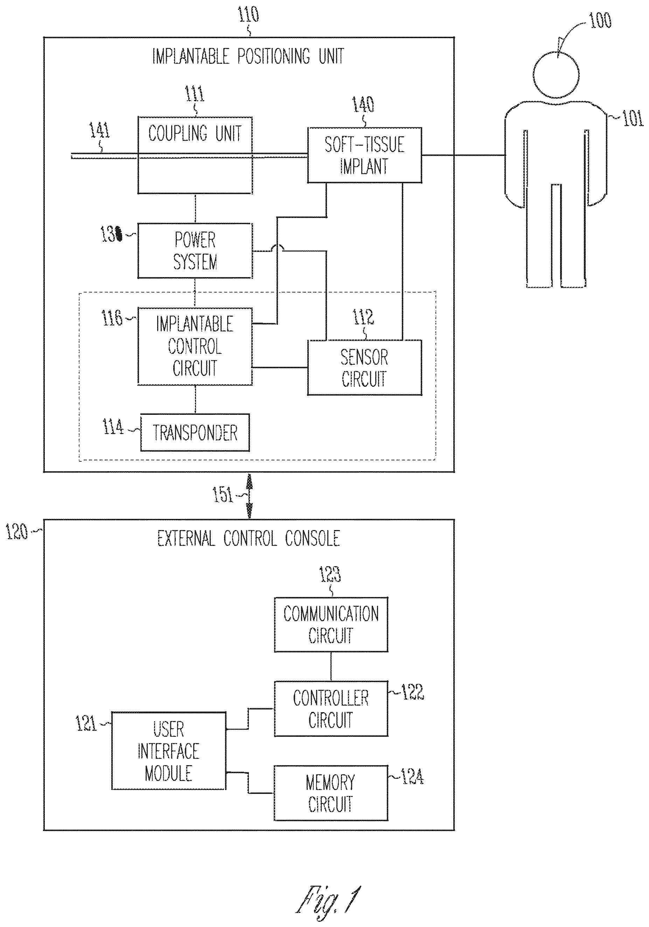

FIG. 1 is a diagram illustrating, by way of example and not limitation, a robotically assisted and dynamically controlled soft-tissue manipulator system 100 and portions of an environment in which the system 100 may operate. The soft-tissue manipulator system 100 may include an implantable positioning unit (IPU) 110 and an external control console 120. The IPU 110 may be completely or partially implantable.

The IPU 110 may include one or more of a coupling unit 111, a sensor circuit 112, a power system 130, a transponder 114, and an implantable control circuit 116. The coupling unit 111 may interface with an elongate member 141. A soft-tissue implant 140 may be coupled to the elongate member 141 such as on a distal end thereof. The coupling unit 111 includes actuating members arranged to engage at least a portion of the elongate member 141, and robotically propel the elongate member 141 to move soft-tissue implant 140 into a target site of a patient 101. Examples of the actuating members may include motorized actuation via rollers, screws, gears, or rack-pinion, among others. In an example, the elongate member 141 may be an integral part of the soft-tissue implant 140, such as a tubular implant body or an elongate or telescoping shaft. Examples of such an implant may include an implantable lead or catheter. Alternatively, the elongate member 141 may be a part of a delivery system detachably coupled to the soft-tissue implant 140. Examples of such an implant may include a guidewire or an introducer that may snatch an implant at a particular location, such as at a distal portion of the elongate member 141.

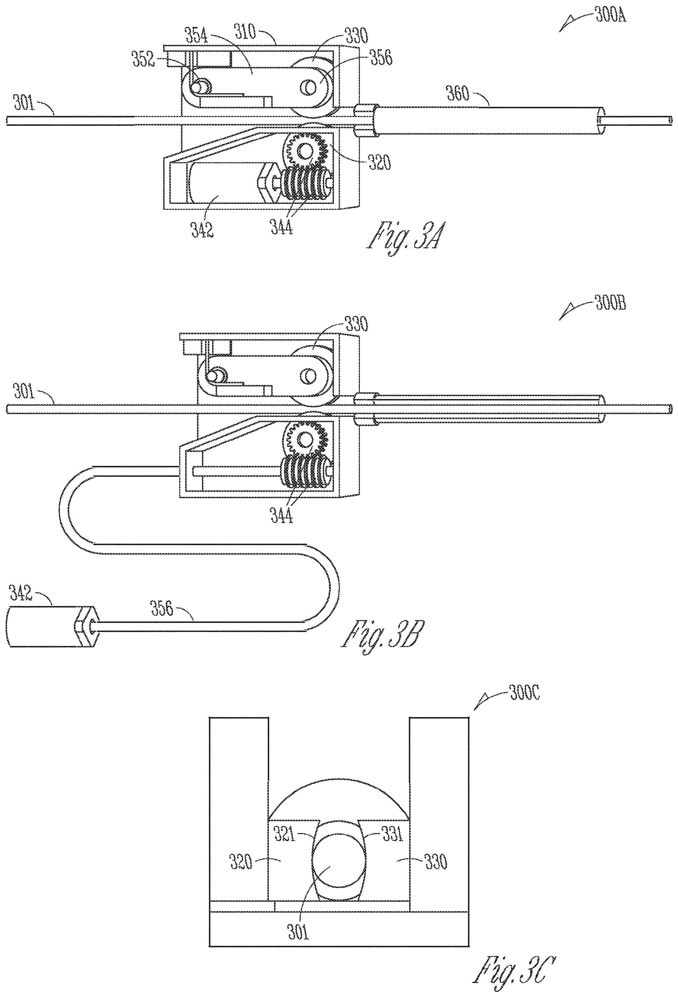

The coupling unit 111 may frictionally move the elongate member 141 to a specific direction (e.g., forward for implant insertion, or reverse for implant extraction), at a specific rate, or for a specific distance relative to a reference point such as the interface between the coupling unit 111 and the elongate member 141. Examples of the coupling unit 111 may include a leadscrew, a clamp, a set of rotors, or a rack and pinion arrangement, among other coupling mechanisms. The coupling unit 111 may compress against at least a portion of the elongate member 141 to produce sufficient friction between the coupling unit 111 and the elongate member 141. In some examples, the coupling unit 111 may include adjustable couplers for reversible or interchangeable connection between the IPU 110 and the elongate member 141. In the event of implant exchange or replacement, the coupling unit 111 may operatively release the compression on the elongate member 141, which may be then removed from the IPU 110. A new implant with an elongate member may be reloaded and engaged into the IPU 110. The IPU 110 need not be removed and may remain in place during implant replacement. Examples of the coupling unit 111 are discussed below, such as with references to FIGS. 3A-3B.

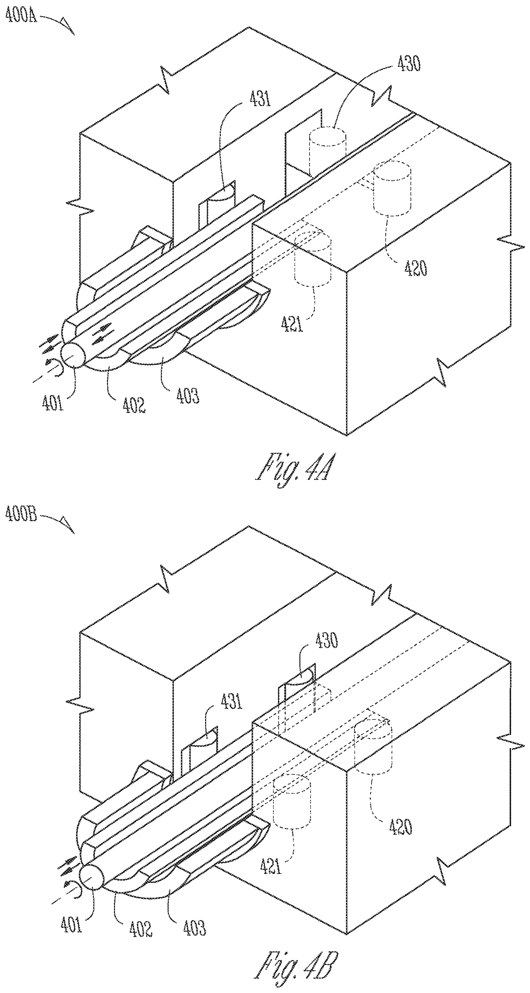

In some examples, the soft-tissue implant 140 may be delivered through a guide sheath. In some examples, the IPU 110 includes separate structures to control a guide sheath separately from the soft-tissue implant 140. In other examples, the guide sheath may be positioned initially by the IPU 110, and the soft-tissue implant 140 implanted through the previously positioned guide sheath. Examples including positioning of a guide sheath are further discussed with reference to FIGS. 4A-4B.

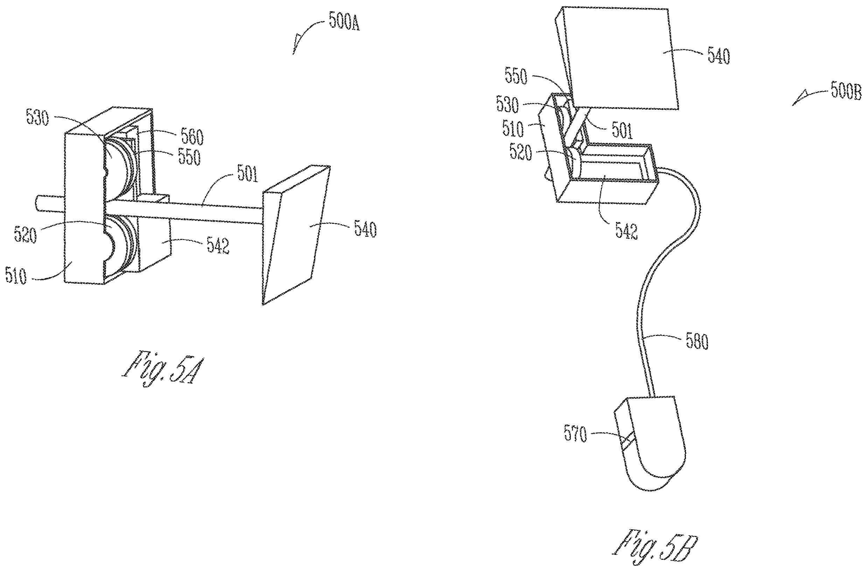

The soft-tissue implant 140 may be delivered and positioned at a target site such that the soft-tissue implant 140 interfaces with target soft tissue. The IPU 110 may manipulate the soft-tissue implant 140 to alter the position or the shape of at least a portion of the target soft tissue. In various examples, the soft-tissue implant 140 may include a soft-tissue prosthesis made of biocompatible material, such as Silastic, goretex, silicon, hydroxyapatite, titanium, or polymer, among other permanent or resorbable materials. In an example, the IPU 110 may be used in a phonosurgery (surgery on the voice box) to address various voice, swallowing, and breathing disorders. A surgeon may robotically control the IPU 110, via the external control console 120 and wireless transponder, to position a thyroplasty implant inside patient voice box to interface with a vocal cord, and manipulating the thyroplasty implant to alter the position and shape of the vocal cord to restore or improve voice. Examples including the thyroplasty implant and adjustment of vocal cord position and shape are discussed below, such as with reference to FIGS. 5A-5B, 6A-6B, and 7A-7B.

Once the soft-tissue implant 140 has been positioned at the target site, the elongate member 141 may be disengaged from the soft-tissue implant 140. Alternatively, the elongate member 141 may remain attached to the soft-tissue implant 140 following the implantation This allows a surgeon to re-optimize implant position in an implant revision procedure following the initial implantation without the need of a surgery to reattach the soft-tissue implant 140 to the elongate member 141.

The power system 130 is configured to provide driving force to the coupling unit 111. The power system 130 includes a motor that may generate driving force and motion, and a power transmission unit to transmit the driving force and motion to the coupling unit 111 to actuate the motion of the elongate member 141. Examples of the motor may include stepper motors (e.g., micro- or nano-stepper motors), direct current (DC) motors, pneumatic or piezoelectric motors, ultrasonic motors, or linear motors, among others. The motor may be electrically coupled to a power source. In an example, the power source may include a rechargeable power source, such as a rechargeable battery or a supercapacitor. The rechargeable power source may be charged wirelessly by a portable device such as a handheld device with circuitry configured to transfer energy to the rechargeable power source through electromagnetic induction or other transcutaneous powering means.

In the example as illustrated in FIG. 1, the power system 130 is at least partially included in or associated with the IPU 110. Alternatively, the power system 130 may be at least partially included in or associated with the external control console 120. In another example, the power system 130 may be separated from the IPU 110 and the external control console 120, and coupled to the coupling unit 111 via a connection. The connection may be a part of the transmission unit.

The implantable control circuit 116 may be coupled to the transponder 114 to receive a motion control signal from the external control console 120. In an example, the coupling between the implantable control circuit 116 and the transponder 114 is a wireless coupling. The motion control signal may specify values for various implant motion parameters, and can be generated according to user programming instructions such as provided via the user interface module 121. The implantable control circuit 116 may control the motor to generate driving force and motion according to the received motion control signal, and drive motion of the elongate member 141 via the power transmission unit and the coupling unit 111. Examples of the power transmission unit may include chains, spur gears, helical gears, planetary gears or gearhead, worm gears, miniature pulleys, shaft couplings, or timing belts, among others. The power transmission unit may adjust the speed or torque output from the motor, and to deliver specific output to the coupling unit 111.

In various examples, the power system 130 may include two or more motors coupled to respective power transmission units, and the power transmission units are coupled to respective coupling units that engage the same elongate member 141 at different locations thereof. The two or more motors may be of the same or different types. The transponder 114 may receive from the external control console 120 a motion control signal for controlling each of the two or more motors. In an example, a user may program and control each of the motors independently, such as via the user interface module 121. The motion control signal specifies the configuration of, and input voltage or current to, each of the motors. According to the motion control signal, the implantable control circuit 116 may control the two or more motors to generate respective torque, speed, or rotation direction Through the elongate member 141, the IPU 110 may operatively move the soft-tissue implant 140, and therefore adjusting the target soft tissue, in multiple axis and planes with up to six degrees of freedom (medial, lateral, superior, inferior, anterior, and posterior). In an example, a first motor produces a translational motion of the elongate member 141, and second motor may produce a rotational motion of the elongate member 141. The implantable control circuit 116 controls various translational motion parameters (e.g., translational rate, direction (advancement or withdrawal), distance relative to a reference point, a position of a distal end of the elongate member 141, an amount of axial force applied to the elongate member 141), and rotational motion parameters (e.g., angular position, angular displacement, angular velocity, or an amount of lateral or rotational force applied to the elongate member 141).

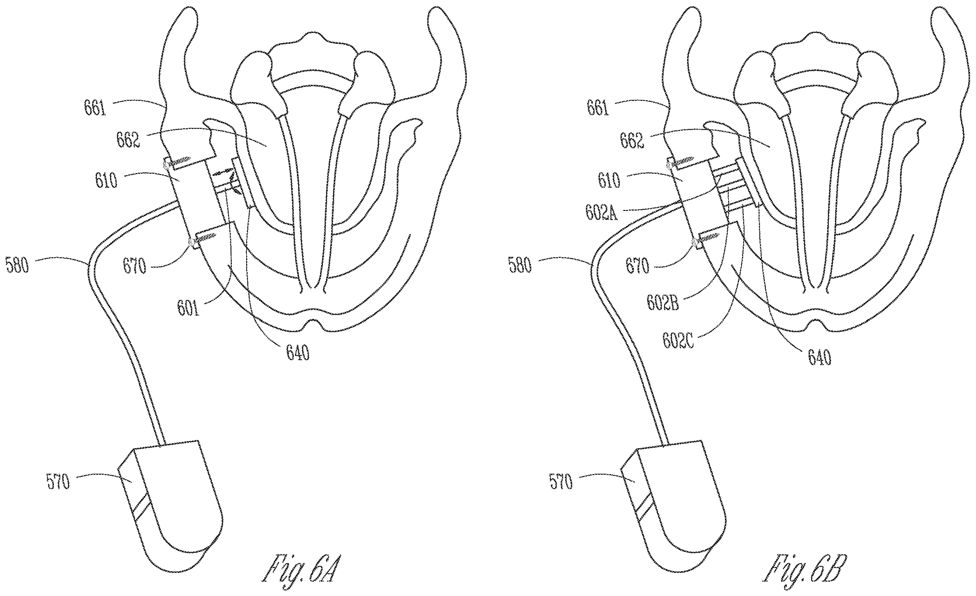

In some examples, the soft-tissue implant 140 may be attached to two or more elongate members each representing an embodiment of the elongate member 141. Each elongate member may be coupled to a respective coupling unit representing an embodiment of the coupling unit 111. The transponder 114 may receive from the external control console 120 a motion control signal for controlling each of the two or more motors. According to the motion control signal, the implantable control circuit 116 may control the two or more motors to generate driving forces to independently move the respective elongate members in different direction (e.g., advancement or withdrawal) or at different rate. Through independent control of multiple elongate members, the IPU 110 may operatively move the soft-tissue implant 140, and therefore adjusting the target soft tissue, in multiple axis and planes. For example, the soft-tissue implant 140 may not only be advanced or withdrawn translationally, but may slant or rotate at different angles, thereby manipulating the target soft tissue at a desired positon or with a desired shape. Examples of positioning and manipulating a soft-tissue implant coupled to multiple elongate members are discussed below, such as with reference to FIG. 6B.

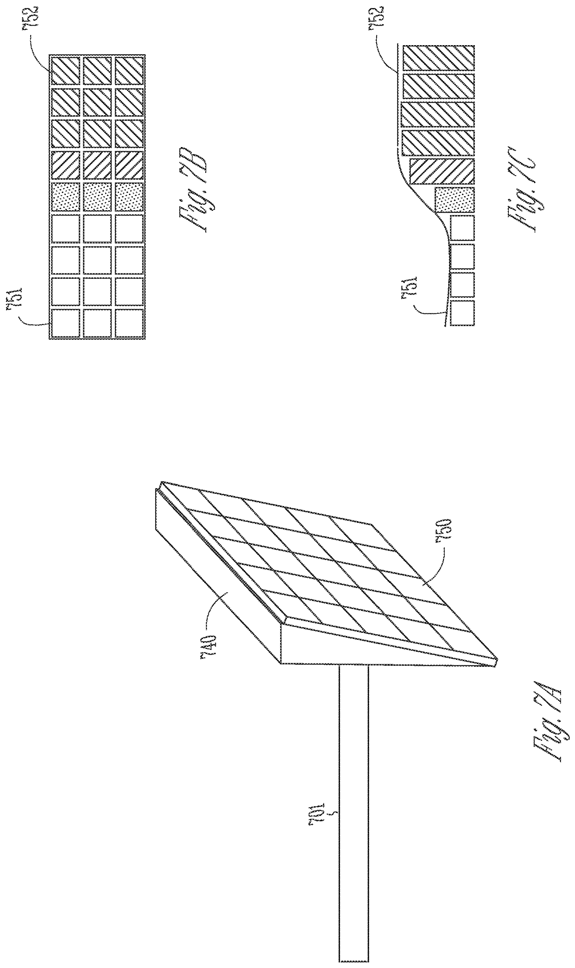

In various examples, the implantable control circuit 116 may change the shape or physical dimension of at least a portion of the soft-tissue implant 140, such as topography of an implant surface interfacing with the target soft-tissue. The soft-tissue implant 140 may include an array of micro-actuators on the tissue-interfacing surface of the implant. In response to an implant contour control signal from the external control console 120, the implantable control circuit 116 may activate the micro-actuators to change tissue-contacting surface contour. The change in the implant shape may result in changes in position or shape of at least a portion of the soft tissue. Compared to the motion control of the soft-tissue implant 140 via the power system 130 and the elongate member 141 for "macro position adjustment" of the target soft tissue, the surface contour control of the soft-tissue implant 140 via the micro-actuators may be used for "micro position adjustment" of the target soft tissue. Examples of controlled change of implant surface contour and the associated micro adjustment of soft tissue position are discussed below, such as with reference to FIGS. 7-8.

The sensor circuit 112 may be configured to sense information about position or motion of the implant during implantation. The sensor circuit 112 may be attached to the motor or the power transmission unit within the power system 130, or associated with the coupling unit 111, to detection information about position of the implant. Examples of the sensor circuit 112 may include a Hall-effect sensor integrated in the motor, one or more optical sensors attached to the coupling unit, a capacitive sensor configured to detect implant motion. The sensor circuit 112 may include force sensors included in the power system 130 or the coupling unit 111, or associated with the soft-tissue implant 140, to sense a parameter indicative of force or friction imposed on the implant during the implant advancement such as axial, lateral, or radial forces when the soft-tissue implant 140 interacts with the target soft-tissue. Examples of the force sensors may include resistors, capacitive sensors, piezoelectric material, or a strain gauge, among others. In an example, the force may be indirectly sensed by measuring the current supplied to the motor. The current measurement may be transmitted to the external control console 120, where it is converted to the force (or torque) using the torque-current curve predetermined and stored in the memory circuit 124. In some examples, the sensor circuit 112 may include sensors on the soft-tissue implant 140 to provide information indicative of shape or contour of the tissue-contacting surface of the implant 140, such as before and after applying voltage to the micro-actuators on the tissue-contacting surface of the implant.

The information acquired by the sensor circuit 112 may be forwarded to the external control console 120 via the communication link 151, The sensor information may be displayed or otherwise presented in a specific media format in the output module 126. In an example, the IPU 110 may include an indicator to produce a visual or audio notification in response to the sensed sensor signal satisfies a specific condition. The indicator 213 may include, for example, a light emitting diode (LED) that may be turned on when the sensed sensor signal indicates the implant reaches the target site. In some examples, the indicator may include a plurality of LEDs with different colors or different pre-determined blinking patterns. The LED colors or the blinking patterns may correspond to various events encountered during the implantation procedure.

The IPU 110 may be configured for subcutaneous implantation. An implantable position device such as the IPU 110 is advantageous in applications such as thyroplasty surgery, which may have a high revision rate following the initial implantation. The IPU 110 allows a surgeon to remotely and dynamically adjust the position of the pre-implanted thyroplasty implant, without the need of surgical intervention, to re-optimize patient vocal quality when the implant status or patient condition changes following the initial implantation. In an example, electrical and mechanical components of the IPU 110 may enclosed in a housing that may be anchored to an anatomical structure neighboring the target soft tissue. In another example, the components of the IPU 110 may be packaged into separate housings that may be implanted at different body locations. For example, the power system 130 and the coupling unit 111 may be enclosed in a first housing to be anchored to thyroid cartilage of the voice box neighboring the vocal cord, while the implantable control circuit 116, the sensor circuit 112, and the transponder 114 may be assembled on a circuit board enclosed in a second housing subcutaneously implanted at a body location away from the vocal box, such as under the skin on the neck or chest. Examples for anchoring the IPU to structures at various body locations are discussed below, such as to be discussed in detail with reference to FIGS. 5A-5B and 6A-6B.

The IPU 110 may include a fixation member to allow for detachable affixation of the IPU 110 to the anchoring structure. The fixation may be invasive fixation that involves incision and/or penetration of the anchoring structure or the subcutaneous tissue. Examples of the fixation member may include one or more of a screw, a pin, a nail, a wire, a hook, a barb, a helix, a suture, a glue, or a magnet within the IPU 110 coupled to one or more magnetic screws or pins affixed to the body part of the patient 101. In an example, the fixation member may include one or more of self-drilling screws, self-tapping screws, or self-piercing screws, such that no pilot hole needs to be drilled at the affixation site prior to screw installation.

In some examples, while some portion of the IPU 110 is implantable, at least a portion of the IPU 110 may be externally positioned, such as a portion of the power system 130 (e.g., power source, or motor), the sensor circuit 112, or the transponder 114, among others. The non-implantable components may be packaged and affixed to the skin of a body part using non-invasive fixation means, such as clamps, temporary glues, or other holding devices that prevent lateral motion relative to the patient 101. The external package may be a compact and lightweight for direct attachment to the patient, such as on the patient neck or check during a thyroplasty implant surgery, while maintaining sufficient stability during the implantation. The external package may be sized and shaped to facilitate patient attachment, such as having a curved exterior surface that conforms to the contour of a body part of the patient 101.

The contact surface of the IPU 110 may be processed to improve stability during the implant advancement procedure. In an example, the IPU 110 may have an exterior surface with a rough finish, such as ridges, corrugates, teeth, or other coarse surface textures. Additionally or alternatively, the IPU 110 may have one or more gripping elements configured to frictionally bond the IPU 110 to a body part of the patient 101, such as the anchoring structure for subcutaneous implantation or epicutaneous placement. The gripping elements may be distributed on a portion of the exterior surface. Examples of the gripping elements may include penetrators such as spikes, pins, or barbs protruding from the exterior surface When the IPU 110 is pressed and held against the attachment region, the rough surface or the gripping elements may provide sufficient friction or gripping force to securely hold the IPU 110 in place relative to the patient 101 during the implantation advancement.

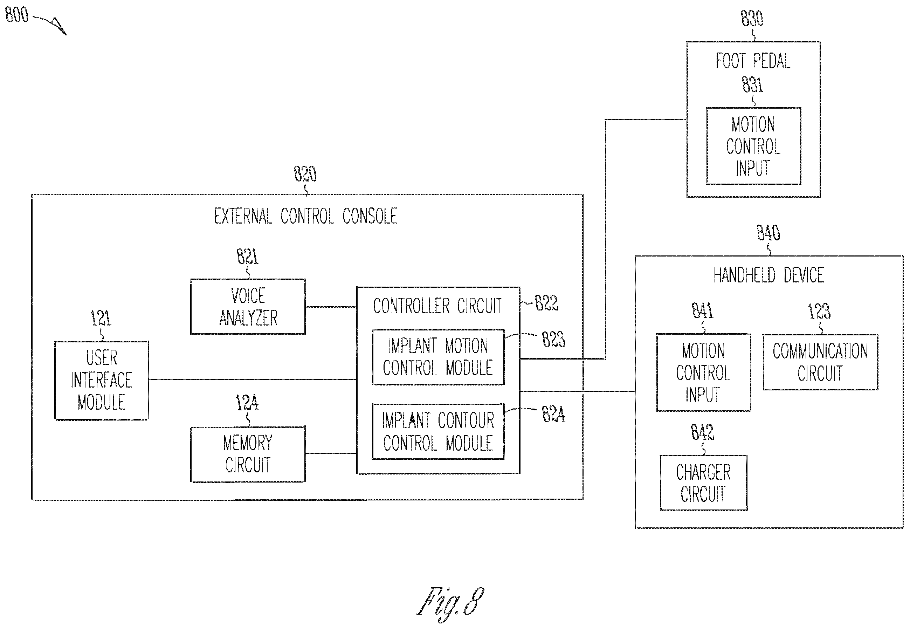

The external control console 120 may include a dedicated hardware/software system such as a programmer, a remote server-based patient management system, or alternatively a system defined predominantly by a controller software running on a standard personal computer. The external control console 120 may robotically control the coupling unit 111 to propel the elongate member 141 at specific rate, to a specific direction, for a specific distance, or at a specific maximum force, thereby positioning the soft-tissue implant 140 at the target site of the patient 181. The external control console 120 may additionally receive information acquired by the sensor circuit 112. The external control console 120 may also receive measurement data from external systems that can be directly related to implant position. The external control console 120 can utilize such measurement data (e.g., physiological measurements) for closed-loop control of implant positioning and manipulation. For example, the external control console 120 may receive patient voice input via the user interface module 121 as feedback to manipulate a thyroplasty implant, and thereby adjusting the vocal cord position and shape, as to be discussed in the following with reference to FIG. 8. In various examples, the external control console 120 may include a physiologic sensor configured to sense a physiologic signal, such as respiration or muscular movement of the patient. The controller circuit 122 may determine dynamic motion control feedback, and control the positioning and manipulation of the implant further using the sensed physiologic signal.

The external control console 120 may include a user interface module 121 and a controller circuit 122. The user interface module 121 may include a user input module and an output module. The user input module may be coupled to one or more input devices such as a keyboard, on-screen keyboard, mouse, trackball, touchpad, touch-screen, or other pointing or navigating devices. In some example, the user input module may be incorporated in a mobile device communicatively coupled to the external control console 120, such as a handheld device. The user input module may be configured to receive motion control instructions from a user. The motion control instructions may include one or more target motion parameters characterizing desired movement of the elongate member 141 of the implant. For example, the target motion parameters may define maximum values or value ranges of the motion parameters. Examples of the target motion parameters may include a target movement rate, a target movement direction or orientation, a target movement distance, a target position of a distal end of the elongate member, or a target amount of force imposed on the elongate member 141. The movement of the implant may be activated at intervals of a predetermined step size. In an example of implantation of a thyroplasty implant the target movement distance may range from 0.1 - 20 millimeter (mm). The target movement rate is approximately at 100-micron intervals. The motion control instructions may include a pre-determined implant delivery protocol that defines target values of a plurality of motion parameters. The implant delivery protocols are designed to ease the programming of the motion control, and to minimize peri-surgical tissue trauma or damage to the surrounding tissue.

The user interface module 121 may allow a user to select from a menu of multiple implant delivery protocols, customize an existing implant delivery protocol, adjust one or more motion parameters, or switch to a different implant delivery protocol during the implant delivery procedure. The external control console 120 may include a memory circuit 124 for storing, among other things, motion control instructions. In an example, one of the delivery protocols may include use of intraoperative physiologic measures that can reflect immediate changes in soft-tissue mechanics and insertion trauma pre-, during-, and post-insertion of the soft-tissue implant 140. In an example of implantation or revision of a thyroplasty implant, the delivery protocols may include use of intraoperative patient voice feedback.

The output module may generate a human-perceptible presentation of information about the implant delivery control, including the programmable motion control parameters, and the motion control instructions provided by the user. The presentation may include audio, visual, or other human-perceptible media formats, or a combination of different media formats. The output module 126 may include a display screen for displaying the information, or a printer for printing hard copies of the information. The information may be displayed in a table, a chart, a diagram, or any other types of textual, tabular, or graphical presentation formats. Additionally or alternatively, the output module 126 may include an audio indicator such as in a form of beeps, alarms, simulated voice, or other sounds indicator.

The output module 126 may also generate presentation of data sensed by the sensor circuit 112, including data such as current position and movement rate of the implant, the force or friction applied to the implant motion. This allows a surgeon to monitor in real-time the progress of the implantation, and adjust the motion control as needed. The presentation may include real-time visual or audible notification with specified patterns corresponding to different types of events encountered during implantation. In an example, the output module 126 may include a visual indicator, such as a light emitting diode (LED) or an on-screen indicator on the display screen. A specific LED color or a specific blinking pattern may signal to the user a successful positioning of the implant at the target site. A different LED color or a different blinking pattern may alert an excessive force imposed on the implant due to unintended tissue resistance during the implant advancement. The output module 126 may additionally or alternatively include an audio indicator, such as a beep with a specific tone, a specific frequency, or a specific pattern (e.g., continuous, intermittent, pulsed, sweep-up, or sweep-down sound). In an example, a beep or an alarm with a specific tone or pattern may signal to the user successful positioning of the implant at the target site. A beep or an alarm with a different tone or different pattern may alert an excessive force imposed on the implant. In an example, the beep or the alarm may go off continuously as the sensor senses the implant approaching the target site. The sound frequency or the pulse rate of the beep or the alarm may increase as the implant gets closer and finally reaches the target site. In an example, the frequency of the beep or the alarm may correspond to a rate of motion, such as sounding for every one millimeter of motion. Audible feedback on the motion parameters may be advantageous in that the surgeon may be notified in real time the implantation status or events encountered without the need to look away from surgical field. This may assist surgeon with enhanced surgical precision and patient safety. In some examples, the audible or visual sensor feedback may signal to the user that the sensed implant position, motion, or for has exceeded the programmed target or maximum parameter values.

The controller circuit 122 may be configured to generate an implant motion control signal and/or an implant contour control signal for controlling the IPU 110 to deliver, position, and manipulate the soft-tissue implant 140. Such control signals may be generated according to the motion control instructions provided by the user via the user input module 125. In accordance with the motion control signal, the implantable control circuit 116 may control the power system 130 to regulate one or more motion parameters of the elongate member 141, such as a movement rate, a movement direction or orientation, a movement distance, a position of a distal end of the elongate member, or an amount of force imposed on the elongate member 141, among others. In some examples, the controller circuit 122 may generate multiple motion control signals that may be used to respectively control multiple motors configured to drive different modes of motion (e.g., translational or rotational motions) on the same elongate member 141, or to drive different elongate members, as discussed above. In some examples, the controller circuit 122 may control the motion of the elongate member 141 further according to information about patient medical history or disease state received via the user input module 125, or stored in the memory circuit 124. In accordance with the motion control signal, the implantable control circuit 116 may activate an array of micro-actuators such as by applying a voltage map to change tissue-contacting surface contour, thereby causing changes in shape or position of the target soft tissue.

The controller circuit 122 may remotely control the IPU 110 via a communication circuit 123. The communication circuit 123 may transmit the motion control signal to the power system 130 via the communication link 151. The communication link 151 may include a wired connection including universal serial bus (USB) connection, or otherwise cables connecting the communication interfaces on the external control console 120 and the power system 130. The communication link 151 may alternatively include a wireless connection, such as a Bluetooth protocol, a Bluetooth low energy protocol, a near-field communication (NFC) protocol, Ethernet, IEEE 802.11 wireless, an inductive telemetry link, or a radio-frequency telemetry link, among others.

In various examples, the IPU 110 may include a manual control mechanism in addition to the robotic control of the coupling unit 111. The manual control mechanism may bypass or override the robotic motion control of the soft-tissue implant 140. Examples of the manual control mechanism may include a dial turn, a screw, or direct insertion technique. The output module 126 may enable a user to selectably enable a robotic mode for robotically assisted motion control via the power system 130, or a manual override mode for manual motion control of the elongate member 141. Alternatively, an operation on the manual control mechanism may automatically withhold or disable the robotic motion control of the elongate member 141.

Portions of the external control console 120 may be implemented using hardware, software, firmware, or combinations thereof. Portions of the external control console 120 may be implemented using an application-specific circuit that may be constructed or configured to perform one or more particular functions, or may be implemented using a general-purpose circuit that may be programmed or otherwise configured to perform one or more particular functions. Such a general-purpose circuit may include a microprocessor or a portion thereof, a microcontroller or a portion thereof, or a programmable logic circuit, or a portion thereof. For example, a "comparator" may include, among other things, an electronic circuit comparator that may be constructed to perform the specific function of a comparison between two signals or the comparator may be implemented as a portion of a general-purpose circuit that may be driven by a code instructing a portion of the general-purpose circuit to perform a comparison between the two signals.

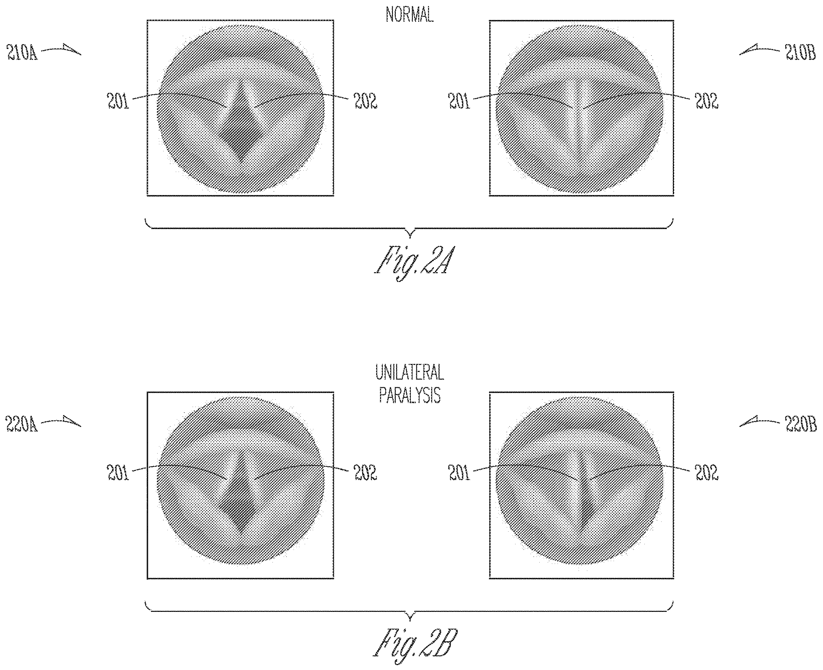

FIGS. 2A-2B illustrate normal vocal cords and those with vocal cord paralysis (VCP), a medical condition that may be treated or alleviated using the robotic soft-tissue manipulator system 100. The vocal cords (also known as vocal folds) are located within the voice box (larynx) at the top of the trachea, consisting of two infoldings 201 and 202 of mucous membrane stretched horizontally, from back to front, across the larynx. The vocal cords 201 and 202 are attached posteriorly to the arytenoid cartilages, and anteriorly to the thyroid cartilage. Normally, as illustrated in FIG. 2A, the vocal cords 201 and 202 remain open when a subject is silent 210A, creating an airway through which one can breathe. When one speaks 210B, the vocal cords 201 and 202 each move towards the middle of larynx, and close the airway. The air from lungs is forced through the closed vocal cords 201 and 202 and cause them to vibrate, which generate sounds.

Opening and closing of the vocal cords are controlled by the vagus nerve. During VCP, nerve impulses to the small muscles controlling the voice box are disrupted, such that one or both of the vocal cords 201 and 202 are unable to move laterally during respiration, or to move medially during speech. FIG. 2B illustrates vocal cords in the case of unilateral VCP, which accounts for most cases of VCP. As an example, one cord 202 is paralyzed but the other cord 201 is normal. The paralyzed cord 202 cannot move laterally during respiration, or move medially during speech to close the airway. The incomplete closure of the vocal cords may cause hoarseness, vocal weakness, swallowing difficulties, and breathing disturbances. The IPU may receive physiologic feedback from implanted sensors such as respiration or swallowing muscle or neural signals to modify implant position in real-time coupled to respirations or swallowing such that the implant lateralizes during respiration to open airway yet medialize during swallowing to close airway temporarily to protect from food or fluid aspiration.

Phonosurgery is a procedure involving surgical repositioning of the paralyzed vocal cord to restore vocal activity, usually with injections or implants into the region of the vocal cords. As to be discussed below, the robotic soft-tissue manipulator system 100 may be used in a phonosurgery, where the soft-tissue implant 140 may be delivered and positioned to interface with the paralyzed cord 202. Through the external control console 120, a surgeon may robotically control the IPU 110 to manipulate the soft-tissue implant 140, and modify the position and/or shape of the paralyzed cord 202 to restore or improve voice quality.

FIGS. 3A-3C illustrate, by way of example and not limitation, diagrams of implantable positioning units (TPUs) 300A and 300B each coupled to an elongate member 301. The IPUs 300A and 300B each represent an embodiment of the IPU 110, and the elongate member 301 represents an embodiment of the elongate member 141.

The IPU 300A illustrated in FIG. 3A includes a housing 310 that encloses electro-mechanical components interconnected to engage the elongate member 301 and robotically deliver and position the implant attached to the elongate member 301 into a target site. The housing 310 may include an entrance and an exit ports to feed the elongate member 301 through the IPU 300A. The IPU 300A may include at least two rollers, such as a drive wheel 320 and an idler wheel 330, which are embodiments of the coupling unit 111 or 211. The drive wheel 320 and the idler wheel 330 are arranged and configured to engage at least a portion of the elongate member 301. The engagement of the elongate member 301 may be through compression between respective radial outer surfaces of the drive wheel 320 and an idler wheel 330.

The drive wheel 320 may be coupled via a bearing to an axle that is securely attached to the housing 310, such that the drive wheel 320 may rotate on the axle without lateral movement relative to the housing 310. The drive wheel 320 may be coupled to a motor 342 via a power transmission unit 344. The motor 342, which is an embodiment of one of the motor 231, may generate driving force and motion according to a motion control signal provided by the external control console 120. The motor 231 may be coupled to the power transmission unit 344, which may be an embodiment of one of the power transmission unit 232. The power transmission unit 344 may include gears, pulleys and belts, or timing belts that adjust a speed or torque of the motors. In an example as illustrated in FIG.3A, the power transmission unit 344 may include a worm gear set 344 comprising a worm gear, and a shaft securely coupled to a gearhead of the motor 342. Depending on the motion control signal input to the motor 342, the power transmission unit 344 may drive rotation of the drive wheel 320, which in turn propels the implant to a specific orientation or at specific rate.

The idler wheel 330 may be coupled to a biasing system that includes a torsion spring 352, a pivot arm 354, and a spring bias 356 interconnected to support the second wheel 330 and to provide lateral compression against the drive wheel 320. The torsion spring 352 may produce spring tension relayed to the second wheel 310 via the pivot arm 354, and compress against the drive wheel 320 to generate adequate friction on the elongate member 301 between the drive wheel 320 and the idler wheel 330. Because the idler wheel 330 is held in place by the biasing system rather than being affixed to the housing 310, the idler wheel 330 may move laterally relative to the housing 310. This may allow for accommodating implants with elongate members of a range of diameters or cross-sectional shapes, while maintaining sufficient friction on the elongate member for desirable movement. In an example, a user may manually bias the torsion spring 352 and move the idler wheel 330 away from the drive wheel 320, thereby release the compression and open the space between the drive wheel 320 and the idler wheel 330. The surgeon may remove the elongate member 301 from the IPU 300A, or load another implant with an elongate member into the IPU 300A.

In some examples, the IPU 300A or 300B may enable manual control over the motion of the elongate member 301. At least one roller, such as the drive wheel 320, may be coupled to a manual drive wheel via a transmission unit, such as a gear set including a spur gear, one or more of chains, belts, or shaft couplings, among others. A user may manually access and rotate the manual drive wheel to drive rotation of the drive wheel 320, and frictionally move the elongate member 301 at a desired direction and speed. In some examples, the manual motion control discussed herein may be combined with the motorized motion control in the IPU 300A or 300B. For example, the drive wheel 320 may be subject to both a robotic control through the motor 342 and the power transmission unit 344, and a manual control through the manual drive wheel and the coupled transmission unit. The robotic control and the manual control may be activated independently from each other. In an example, the user interface module 121 may enable a user to select between a robotic mode for robotic motion control and a manual mode for manual motion control of the elongate member 301. In an example, the manual mode may take priority over or automatically override the robotic mode. The manual override function may be utilized as a fail-safe emergency stop in case of a fault in the motor 342 or the power transmission unit 344.

In some examples, the radial outer surface of the drive wheel 320 may be coated with a frictious material, such as a layer of silicone rubber, polymer, or other composite materials. Additionally or alternatively, the radial outer surface of the drive wheel 320 may be mechanically textured to have a rough and corrugated surface. The frictious material layer or the corrugated surface finish of the radial outer surface of the drive wheel 320 may increase the friction and prevent the elongate member 301 from slipping on the drive wheel 301 during frictional motion. The radial outer surface of the idler wheel 330 may similarly be coated with the frictious material or have a rough surface finish.

Although motorized rollers (including the drive wheel 320 and the idler wheel 330) are discussed herein, this is mean to be an illustration rather than a limitation. Other actuating members such as motorized screws, gears, or rack-pinion may alternatively or additionally be used in the systems, apparatus, and methods discussed in this document.

FIG. 3C illustrates a cross-sectional view 300C of the drive wheel 320 and the idle wheel 330 with the elongate member 301 engaged therebetween. In an example as illustrated in 300C, the elongate member 301 has a cylindrical shape or otherwise has a convex cross-sectional profile. The radial outer surface 321 of the drive wheel 320 and the radial outer surface 331 of the idle wheel 330 may each have a radially concave profile to allow for secure engagement of the elongate member 301. The concavity of the concave profile, which quantifies a degree of the concave surface, may be determined based on the geometry such as the diameter of the elongate member 301.

The drive wheel 320 and the idler wheel 330 illustrated in FIG. 3A may generate one-degree of freedom of movement, such as a translational motion. In some examples, the IPU 300A may include additional wheels or gear sets arranged and configured to translate the force and motion generated from the motor 342 into multiple-degrees of freedom movement, as previously discussed with reference to FIG.2. In an example, the IPU 300A may include a gear set to translate the motor motion into a rotational motion of the elongate member 301 around its axis. The gear set may include a geared drive wheel coupled to a worm gear coaxially disposed along, and detachably coupled to, a portion of the elongate member 301. The geared drive wheel, when driven to rotate by the motor 342 and the power transmission unit 344, may drive rotation of the worm gear, which in turn cause the rotation of the elongate member 301 around its axis.

The drive wheel 320 and the spring-biased idler wheel 330 are an example of the coupling unit by way of illustration and not limitation. An alternative coupling unit may include a geared drive wheel coupled to an implant carrier. The carrier may include an adapter housing placed over and securely hold the elongate member of the implant. The adapter housing may be made of silicone or metal. The carrier may have a linear gear arrangement with teeth configured to engage with the geared drive wheel. The geared drive wheel and the linear gear of the carrier may thus have a rack-and-pinion arrangement, where the geared drive wheel (the pinion) applies rotational motion to the linear gear (the rack) to cause a linear motion relative to the pinion, which in turn may linearly move the elongate member held within the adapter housing of the carrier.

One or more sensors may be attached to the internal components of the IPU 300A, such as the motor 342, the power transmission unit 344, the drive wheel 320, or the spring-biased idler wheel 330. Examples of the sensors may include an encoder or a Hall-effect sensor. The sensors may sense the location or motion of the elongate member 301, or the force or friction applied to the elongate member 301. In an example, a first sensor may be attached to the motor 342 to detect the motion of the motor (which indicates the position or motion of the elongate member 301), and a second sensor may be attached to the idler wheel 330 to detect the motion of the idler wheel (which also indicates the position or motion of the elongate member 301). The first and second sensors may jointly provide a double check of the implant's position, and can more reliably detect any slippage that may occur between the drive wheel 320 and the elongate member 301. For example, if the motor 342 functions normally but the elongate member 301 slips on the drive wheel 320, the first position sensor on the motor would indicate implant movement, but the second position sensor on the idler wheel 330 would indicate no movement or irregular movement of the implant. The external control console 120 may include circuitry to detect a discrepancy between the position or motion feedbacks from the first and second sensors. If the discrepancy exceeds a specific threshold, the external control console 120 may generate an alert of device fault and presented to the user via the output module 126, or automatically halt the implantation procedure until the user provides instructions to resume the procedure.

The IPU 300A may include a sheath 360. The sheath 360 may be attached to a distal end of the housing 310, and extend to a surgical entrance of the target site. The elongate member 301 may be flexible and prone to twisting, entanglement, or buckling. The sheath 360 may at least partially enclose the elongate member 301 to provide resilient support to the elongate member 301 of the implant, thereby keeping the implant on track between the housing 310 and the surgical entrance of the target site. It may also protect electronics such as an electrode array positioned on the elongate member 301 and the conductors inside the elongate member 301.

The sheath 360 comprises a flexible tube whose dimensions may substantially match the elongate member 301. For example, the diameter of the tube may be slightly greater than the diameter of the elongate member 301, such that the flexible tube may provide desired rigidity to the elongate member 301 inside; while at the same does not produce undue friction between the elongate member 301 and the interior surface of the tube. To decrease friction produced by the motion of the elongate member 301 relative to the tube during implantation, the sheath 360 may be pre-lubricated with a biocompatible and sterilizable lubricant. Alternatively or additionally, the interior surface of the tube may be treated with Polytetrafluoroethylene (PTFE) or linear longitudinal ridges to allow for smooth sliding of the elongate member 301 inside the tube.

The distal end of the sheath 360 may be fixed or reversibly stabilized at a designated position of the surgical opening of the implantation. The sheath 360 may be made of material with low friction, such as plastic or silicone rubber, and biocompatible for tissue contact and compatible with various disposable sterilization methods such as radiation (e.g., gamma, electron beam, or X-ray), or ethylene oxide gas treatment. The sheath 360 may be detached from the implant once the implant is positioned at the target site of implantation. In an example, the sheath 360 may be composed of two longitudinal halves may be connected with a biocompatible and sterilization-resistant adhesive or sealant. The adhesive or sealant may have an adhesion strength sufficient to hold the two longitudinal pieces together, and may be weakened under a pulling stress. In another example, the disengagement means include peel-away sheath with linear perforations on opposing longitudinal sides to facilitate the tearing of the introducer sheath into two opposing pieces.

In some examples, the IPU 300A may be affixed to the patient or an object in the sterile field of surgery. The components inside the IPU 300A, including the drive wheel 320, the idler wheel 330, the idler wheel biasing system (including the torsion spring 352, the pivot arm 354, and the spring bias 356), and the power system (including the motor 342 and the power transmission unit 344), may be made of materials that are both biocompatible and compatible with a specific sterilization method, such as gamma or ethylene oxide. The electro-mechanical components may be made of plastic such as Acrylonitrile-Butadiene-Styrene (ABS), Polycarbonate, Polyetheretherketone, or Polysulfone, among others. The electro-mechanical components may alternatively be made of metal such as stainless steel, cobalt chromium, or titanium, among others.

The IPU 300B as illustrated in FIG. 3B has a similar structure to the IPU 300A, except that the motor 342 is positioned outside the housing 310. The force and motion generated from the motor 342 may be transmitted to the drive wheel 320 via a flex rotating shaft 356 running between the motor 342 and the IPU 300B. In an example, the motor 342 may be enclosed in standalone housing separated from the IPU 300B and the external control console 120. In another example, the motor 342 may be included in or associated with the external control console 120. The flex rotating shaft 356 may be integrated with a communication cable linking the IPU 300B and the external control console 120, such that a single cable exits the positioning unit 300B. The communication cable may transmit the sensor feedback on the position or motion of the elongate member 301, or the forces imposed on the elongate member 301 such as sensed by one or more sensors on the positioning unit 300B, such as illustrated in FIG. 2.

With the exclusion of the motor 342, the IPU 300B may offer several benefits. The IPU 300B may be a smaller, simpler, light-weighted, and low-cost micromechanical device. As the motor 342 and associated electrical system are away from direct patient contact and outside of the patient immediate environment, the IPU 300B may offer an increased patient safety. The IPU 300B may be for single use in a sterile surgical field, and is disposable after the surgery. At least due to its small size and lightweight, the IPU 300B may be suitable for fixation on a patient as a stable platform for advancing the implant.

FIGS. 4A-4B illustrate, by way of example and not limitation, a portion of implantable positioning units (IPUs) 400A and 400B each configured to deliver and position a guide sheath and an elongate member 401. The IPUs 400A and 400B expound on the IPUs 300A or 300B illustrated in FIGS. 3A-3B, and can handle positioning of both an elongate member as well as a guide sheath. As illustrated in FIGS. 4A-4B, the IPUs 400A and 400B each include a guide sheath 403 that can be fixed to the respective IPU. Within the guide sheath 403 is an insertion sheath 402 (also referenced as an internal sheath) and the elongate member 401. Compared to the IPUs 300A and 300B, the IPUs 400A and 400B each include two sets of drive and guide wheels, including spring-loaded sheath wheel 431 and sheath drive wheel 421 and spring-loaded electrode wheel 430 and drive wheel 420. The use of the guide sheath 403 and the insertion sheath 402 serves to reduce the magnitude and frequency of both insertion pressure and insertion forces during the implantation and manipulation of the soft-tissue implant, thereby avoiding trauma to the target soft tissue that may be introduced by manual, un-assisted implantations.

The guide sheath 403 may support and internally house an insertion sheath 402 in a telescoping fashion. The insertion sheath 402 can slide within the guide sheath 403 and over the electrode elongate member 401 also housed within. The insertion sheath 402 moves through the guide sheath 403 (affixed to the IPU proximally) and over the elongate member 401 on the abluminal side within. This enables controlled robotic movement of both the insertion sheath 402 and elongate member 401 into the delicate implantation site.

FIG. 4A illustrates the insertion sheath 402 engaged with spring-loaded sheath wheel 431 and sheath drive wheel 421, which control positioning of the insertion sheath 402. The elongate member 401 is engaged by spring-loaded electrode wheel 430 and electrode drive wheel 420. In this example, the insertion sheath 402 may be fully positioned, as the IPU 400A is engaged with the elongate member 401. FIG. 4B illustrates the insertion sheath 402 engaged with both drive mechanisms within the IPU 400B. The spring-loaded sheath wheel 431 and sheath drive wheel 421, as well as the spring-loaded electrode wheel 430 and electrode drive wheel 420, are engaged with the insertion sheath 402. In this example, the insertion sheath 402 is still in the process of being positioned/delivered.

The IPUs 400A and 400B may be capable of parallel telescoping or rotational movements of both insertion sheaths 402 and 403 and the elongate member 401 in a coordinated, surgeon controlled fashion utilizing two or more coupling units within the IPU. There may either be two independent drive wheel coupling systems each controlling the sheath and electrode insertion independently or in parallel, coordinated motions. After the end of the internal insertion sheath is inserted a distance that passes the distal coupling unit and travels out of the compressive grasp, the spring-loaded wheel will disengage from the internal insertion sheath and clamp onto the internal electrode implant. The now directly interface implant is then controlled robotically with the same drive wheel control unit via user controlled motion parameters.

FIGS. 5A-5B illustrate, by way of example and not limitation, portions of implantable positioning units (IPUs) 500A and 500B configured to position and maneuver a thyroplasty implant 540 to modify the vocal cord position or shape. The IPUs 300A and 500B, which represent embodiments of the IPU 110 and expound on the IPUs 300A or 300B illustrated in FIGS. 3A-3B, may each be used in an initial phonosurgery of implantation, or in a revision procedure following the initial implantation. Modification of the vocal cord position or shape may help restore or improve voice quality in patients suffering from vocal cord paralysis, presbylaryngis, or other chronic voice disorders.

The IPU 500A as illustrated in FIG. 5A includes an electro-mechanical assembly enclosed in a housing 510. The housing 510 may be made of a biocompatible material, such as Silastic, goretex, biocompatible metals or polymer. The housing may be attached to a base configured to be affixed to an anatomical structure, such as thyroid cartilage of the voice box, using a fixation member such as screws, pins, nails, wires, hooks, a suture, or a magnet. Similar to the IPUs 300A or 300B, the illustrated portion of the IPU 500A comprises at least a drive wheel 520 and an idler wheel 530 arranged and configured to engage at least a portion of an elongate member 501, such as through compression between respective radial outer surfaces of the two wheels 520 and 530. The IPU 500A includes a motor 542 that represents an embodiment of the motor 342. In an example, the motor 542 is a piezo stepper motor. The motor 542 is electrically connected to a power source, and may generate driving force and motion according to a motion control signal. The motor 542 is mechanically coupled to a power transmission unit to transmit the force and motion to the drive wheel 520. In this example, the power source, such as a rechargeable battery or a supercapacitor, may be enclosed in the housing 510. The implantable power source may be charged wirelessly such as through electromagnetic induction or other transcutaneous power means.

Enclosed in the housing 510 may include a controller unit 560, which can be implemented on a circuit board that includes one or more of the implantable control circuit 116, the sensor circuit 112, and the transponder 114. The sensor circuit is coupled to an encoder sensor 550 configured to sense rotation of the idler wheel 530 and to measure various motion parameters associated with the elongate member 501, such as position, distance, or force or friction imposed on the thyroplasty implant 540. Examples of the encoder sensor may include optical, capacitive, inductive, or Hall-effect based sensors.

The thyroplasty implant 540 may be made of biocompatible material, such as such as Silastic, goretex, silicon, hydroxyapatite, titanium, or polymer, among other permanent or resorbable materials. The thyroplasty implant 540 may be attached to the elongate member 501, such as a distal end of the elongate member 501. The attachment may be detachable, such that once the soft-tissue implant 140 has been positioned at the target site, the elongate member 501 may be disengaged from thyroplasty implant 540. The detachable design may also allow another elongate member similar to the elongate member 501 to attach to a previously implanted thyroplasty implant 540. Alternatively, the thyroplasty implant 540 may remain attached to the elongate member 501 following the implantation. This allows a surgeon to adjust a pre-existing thyroplasty implant to re-optimize the vocal cord position or shape in a post-implantation revision procedure, yet without the need to reattach the thyroplasty implant 540 to the elongate member 501.

In the illustrated example, the thyroplasty implant 540 has a wedge shape to conform to the orientation of the vocal cord relative to the positon of the anatomical structure to which the IPU 550A is anchored. The wedge shape may improve tissue contact and flexible tissue manipulation. The thyroplasty implant 540 may have an array of piezoelectric actuators with the ability to change shape and contour as needed, as to be discussed in the following in reference to FIGS. 7A-7C. The controller unit 560 may monitor the position, motion, shape, or contour of the thyroplasty implant 540, such as via sensors on the thyroplasty implant 540 or enclosed in the housing 510, and controllably adjust the contour of the tissue-contacting surface of the thyroplasty implant 540 in response to the implant contour control signal received from the external control console 120.

FIG. 5B illustrates by way of example an alternative IPU design. Instead of having an implantable power source and the controller unit 560 enclosed in the housing 510, the IPU 500B includes a second housing 570 separated from the housing 510. Enclosed in the housing 570 include, among other components, a power source (e.g., batteries or supercapacitors) and a control unit such as the control unit 560. The second housing 570 may be electrically coupled to the first housing 510 via a communication link 580, such that the power source and the controller unit 560 may be electrically connected to the motor 542 and the piezoelectric actuators on the thyroplasty implant 540. In an example, the communication link 580 may include wires coated with silicon or other biocompatible materials. The two housings 570 and 510 may be implanted at different body locations. For example, the housing 510 may be anchored to thyroid cartilage of the voice box, and the second housing 570 may be subcutaneously implanted on a neck or chest site.

FIGS. 6A-6D illustrate, by way of example and not limitation, diagrams of portions of IPU 610 for positioning and manipulating a thyroplasty implant 640. The IPU 610 may be anchored into a surgically created window through the thyroid cartilage 661, outside the vocal cords. FIG. 6A illustrates the IPU 610 configured to robotically control position of the thyroplasty implant 640. The IPU 610 includes a motor and a power transmission system to move a single elongate member 601 coupled to the thyroplasty implant 640. The IPU 610 is electrically connected to the subcutaneously implantable housing 570 that encloses a power source and a control unit, as discussed above with reference to the systern 500B. The shape of the thyroplasty implant 640 as it projects into the surgically created window can be oblique with more projection posteriorly than anteriorly. This is to address the average amount of induced medial projection of the vocal cord, which can be approximately 2 mm anteriorly and 6 mm posteriorly. The thyroplasty implant 640 may be positioned such that it interfaces with the target vocal cord 662, such as a paralyzed vocal cord. The IPU 610 may robotically adjust the position and shape of the thyroplasty implant 640, thus push (medialize) the paralyzed vocal cord 662 toward the middle of the vocal box to improve vocalization, or pull (lateralize) the paralyzed vocal cord 662 farther away from the middle of the vocal box to weaken vocal cord closure or to enlarge glottis aperture to improve airway opening and ventilation.

The IPU 610 includes a set of motors configured to provide various modes of motion of the elongate member 601, including translational advancement or withdrawal. and rotational motion such as flexion and extension. FIG. 6B illustrates the IPU 610 that engages multiple elongate members, such as 602A-602C. The IPU 610 may include a set of electric motors configured to independently drive motion of respective elongate members 602A-602C, such as at different directions (e.g., forward or backward) and/or with different speeds. With the independent control of multiple elongate members, the thyroplasty implant 640 may not only move linearly as a whole, but may also slant or rotate at different angles, thus increasing the flexibility of altering the vocal cord position and conformation.

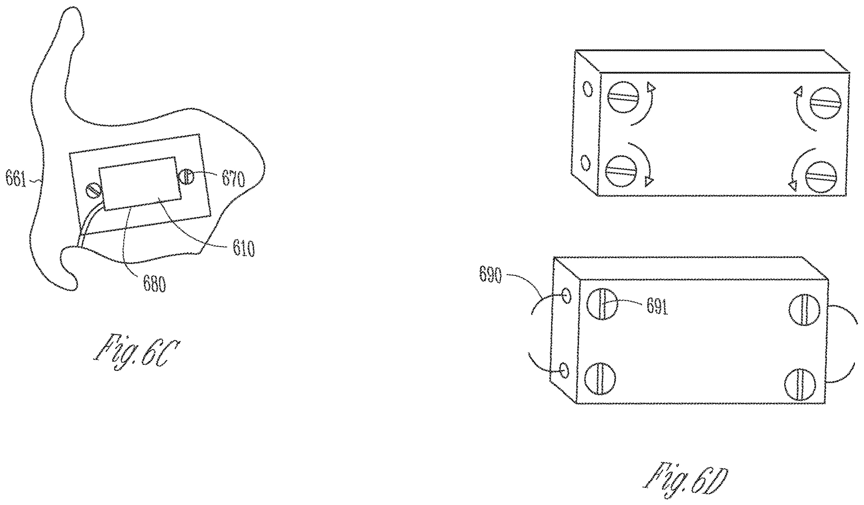

The IPU 610 may be affixed to the thyroid cartilage 661 using affixation means, such as one or more screws 670. Other fixation means may also be used, such as one or more of a pin, a nail, a wire, a hook, a barb, a helix, a suture, a glue, or a magnet within the IPU 610 coupled to one or more magnetic screws or pins affixed to the body part of the patient 101. FIG. 6C illustrates a diagram of affixing the IPU 610 on the thyroid cartilage 661 further using a surgical mesh 680 permitting suture fixation to the cartilage adjacent to the window, such as to provide further support and stabilization of the IPU 610. The surgical mesh 680 may be made of polypropylene, polymer, goretex, Teflon, or titanium, among other biocompatible materials. In various examples, the fixation means may include one or more of self-drilling screws, self-tapping screws, or self-piercing screws, such that no pilot hole needs to be drilled at the affixation site prior to screw installation. FIG. 6D illustrates a diagram of self-piercing curved projections 690 such as barbs or helices that can be extended from one or more outer surfaces of the IPU 610, and pierce through the thyroid cartilage 661 to support and stabilize the IPU 610 on the anchoring cartilage. By way of example and not limitation, the self-piercing curved projections 690 may be coupled to respective screws 691. A user may rotate the respective screws 691, such as by using a screwdriver, to cause projection or retraction of the curved projections.

The IPU 610 and the associated thyroplasty implant 640 may be used to control the positioning of the vocal cord 662 not only during initial implantation, but also in revision procedures without further surgical incision. In the event of device exchange, the elongate member 601 may be disengaged from the IPU 610, and the IPU 610 may be removed from the thyroid cartilage 661. The thyroplasty portion, including the elongate member 601 and the attached thyroplasty implant 640 may remain in place. A new IPU may be surgically implanted, affixed to thyroid cartilage 661, and reconnected with the elongate member 601 and the thyroplasty implant 640.

FIGS. 7A-7C illustrate, by way of example and not limitation, a soft-tissue implant 740 having an array of micro-actuators that can modify position and shape of a target soft tissue. The soft-tissue implant 740 is an embodiment of the soft-tissue implant 140 illustrated in FIG. 1, and may be coupled to an IPU, such as one of the IPUs 300A-300B or 500A-500B, via an elongate member 701. A surgeon may use the IPU to controllably modify the position and shape of a paralyzed vocal cord in a phonosurgery.

As illustrated in FIG. 7A, the soft-tissue implant 740 may include an array of piezoelectric, pneumatic, or hydraulic micro-actuators 750 configured to change the contour of at least a tissue-contacting surface of the soft-tissue implant 740. The micro-actuators 750 may be securely attached to the target soft-tissue such that the target soft tissue may be repositioned in different directions, such as medialization and future lateralization of a vocal cord as discussed above in reference to FIGS. 6A-6B. In an example, the attachment of the micro-actuators 750 to the target soft-tissue is achieved using suture holes or other active fixation mechanisms. In another example, the micro-actuators 750 may be enclosed or encapsulated in a biocompatible material that is capable of tissue ingrowth and integration. The capacity of the soft-tissue implant 740 to not only push (medialize) the vocal cord but also to pull (lateralize) and to shape the vocal cord provides a large number of applications for thyroplasty including but not limited to glottic incompetence arising from vocal cord paralysis. Additionally, the soft-tissue implant 740 may be used as a tissue expander to provide gradual expansion of the vocal cord attended by stimulated cellular growth will permit remodeling of scarred and distorted vocal cord tissue to improve its vibratory capacity and voicing result.

In accordance with an implant contour control signal, the micro-actuators 750 may be actuated to change the tissue-contacting surface contour. The change of the tissue-contacting surface contour may cause changes of the position or shape of at least a portion of the target soft tissue. In an example, the micro-actuators 750 are an array of piezoelectric actuators that may be powered via an implantable power source, such as one included in the IPU (e.g., enclosed in the housing 510), or a power source enclosed in a separate subcutaneously implanted housing (such as the housing 570). Alternatively, the micro-actuators 750 may be powered transcutaneously such as via inductive means. In case of piezoelectric actuators, in accordance with an implant contour control signal, a voltage map specifying voltages for the respective piezoelectric actuators may be generated and applied to respectively to the piezoelectric actuator array 750. Physical dimensions of the piezoelectric actuator array 750 may change in proportion to the applied voltage. Similarly, the hydraulic or pneumatic pressures can be controlled to change in proportion to applied commands. As a result, a unique contour or topography may result on the tissue-contacting surface of the soft-tissue implant 740. The soft-tissue implant 740 is capable of conformational changes in multiple axis and degrees of freedom (anterior, posterior, medial, lateral). FIGS. 7B and 7C illustrate respectively a top view and a side view of a portion of the piezoelectric actuator array 750 when different voltages are applied to individual piezoelectric actuators. In this example, actuators 751 at one region of the surface less deformed than actuators 752 at another region of the tissue-contacting surface. Such a change in physical dimension or topography of the piezoelectric actuator array 750 accordingly change the shape and position of the target soft-tissue interfacing with the piezoelectric actuator array 750. In an example of vocal cord modification, the implantable control circuit may dynamically change the applied voltage, thereby modifying the physical dimensions of the implant surface. As such, a multidimensional surface topography can be defined by the user (e.g., a surgeon) in order to optimize vocal cord position and shape, and voice quality to match the contralateral healthy vocal cord points of contact.

FIG. 8 illustrates, by way of example and not limitation, a block diagram of a portion of an external control system 800 to control an IPU to robotically position and manipulate a soft-tissue implant, such as a thyroplasty implant for modifying position and shape of a vocal cord. The external control system 800 comprises an external control console 820 coupled with one or more peripheral devices for implant motion control. The external control console 820, which represents an embodiment of the external control console 120 illustrated in FIG. 1, may include a user interface module 121, a memory circuit 124, a voice analyzer 821, and a controller circuit 822. These circuits may, alone or in combination, perform the functions, methods, or techniques described herein. In an example, hardware of the circuit set may be immutably designed to carry out a specific operation (e.g., hardwired). In an example, the hardware of the circuit set may include variably connected physical components (e.g., execution units, transistors, simple circuits, etc.) including a computer readable medium physically modified (e.g., magnetically, electrically, moveable placement of invariant massed particles, etc.) to encode instructions of the specific operation. Alternatively, the external control console 820 may be implemented as a part of a microprocessor circuit, which may be a dedicated processor such as a digital signal processor, application specific integrated circuit (ASIC), microprocessor, or other type of processor. Alternatively, the microprocessor circuit may be a general purpose processor that may receive and execute a set of instructions of performing the functions, methods, or techniques described herein.

The controller circuit 822, which represents an embodiment of the controller circuit 122, may include an implant motion control module 823 for macro soft tissue position adjustment, and an implant contour control module 824 for micro soft tissue position adjustment. The implant motion control module 823 is configured to generate an implant motion control signal to control the power system of the IPU to regulate movement rate, a movement direction or orientation, a movement distance, a position of a distal end of the elongate member, or an amount of force imposed on the elongate member 141, among others. The implant contour control module 824 is configured to generate an implant contour control signal for controlling the piezoelectric actuator array such as by applying a voltage map to change tissue-contacting surface contour, thereby causing changes in shape or position of the target soft tissue.

In an example, the controller circuit 822 may receive real-time remote sensor data transmitted from the IPU and the soft-tissue implant, and dynamically control the macro- and micro-positioning of the soft-tissue implant using a feedback-control method. For example, the sensor feedback analyzer 821 may receive sensor information sensed by the sensor circuit 112 within the IPU 110, including position or motion of the soft-tissue implant during the implantation and positioning process, and force or friction imposed on the soft-tissue implant. The sensor feedback analyzer 821 may additionally receive sensor information about the shape, contour, or topography of the tissue-contacting surface of the soft-tissue implant, such as before and after applying voltage to the micro-actuators on the tissue-contacting surface of the implant. The sensor feedback analyzer 821 may then adjust the implant motion control signal or the implant contour control signal based on one or more of sensor feedback including physiologic respiratory and swallowing signals from muscle or neural feedback sensors.

The voice analyzer 821 may be configured to analyze patient voice quality during the implantation or revision procedure when the patient is instructed to vocalize during the procedure. A microphone coupled to the interface module 121 may acquire patient voice, which is analyzed by the voice analyzer 821 to provide an indication of improvement in voice quality. indication of voice quality improvement may be used as feedback by the controller circuit 822 to generate the motion control signal to adjust the position and motion of the soft-tissue implant, and/or to generate the implant contour control signal to adjust the contour and topography of the tissue-contacting surface of the soft-tissue implant, thereby modifying the position and shape of the target soft-tissue.

The peripheral devices may include one or more of a foot pedal 830, or a handheld device 840. In an event that the motor and the power system are included within the IPU, the one or more peripheral devices may be communicatively coupled to the IPU to directly control the motor output. The one or more peripheral devices may be communicatively coupled to the controller circuit 822 of the external control console 820, such as via a wired connection or a wireless communication link. Compared to the external control console 820, the peripheral devices may have smaller size, lighter weight, and more mobility, thereby may provide enhanced operation flexibility. Some peripheral devices, such as a foot pedal, may be reusable. The materials need not be sterilizable or biocompatible to the level at which the IPU materials do.

The foot pedal 830 may provide the surgeon with the means to control the motion of the implant. The foot pedal may be positioned under the patient table, accessible to the surgeon. The foot pedal 830 may comprise a motion control input 831. In an example, the motion control input 831 may include two or more pedals for use to control lead motion at different directions, such as one pedal to activate forward advancement motion, and another pedal to activate retraction motion to fine-tune the implant position or for implant extraction. In another example, the motion control input 831 may include two or more pedals for use to control lead motion at different lead orientation, such as one pedal to control the translational motion, and another pedal to control the rotational motion. In yet another example, the motion control input 831 may include one pedal used for controlling implant advancement, and another pedal used for resetting the current implant position (i.e., setting the current position to zero). If a retraction action is needed, this may be an input on the external control console 820, where the retraction command may be generated from the external control console 820. This would prevent accidental retraction of the implant by stepping on the wrong pedal.

In some examples, each foot pedal may be incorporated with one or more command buttons or switches that are programmed for different functions, such as for controlling various motion parameters including motion rate, motion distance, or amount of force applied to the implant during insertion. In an example, different motion control actions may correspond to programmed duration when pedal is pressed and held, or patterns of the pedal press (such as one press, double press, or a combination of short and long press). For example, a short press may set the current implant position to zero (i.e., position reset), and a long press (e.g., press and hold for at least three seconds) may advance the implant. In an example, one press or button push may correspond to a specific distance of movement, such as 100 microns during an implantation procedure. In another example, the rate of insertion or the distance of the movement may vary based on a degree of foot pedal displacement up to the maximum set insertion rate and distance as programmed by a user via the user interface module 121.

The handheld device 840 may include a motion control input 841, such as buttons, switches, or other selection and activation mechanisms to control one or more motion parameters of the implant. As illustrated in FIG. 8, the communication circuit 123 may be implemented inside the handheld device 840. In an example, the communication circuit 123 may communicate with the IPU 110 via a wireless communication link, including transmitting the motor control signal to the motor 231, and receive sensor feedback from one or more sensors located at the power system 230 or the IPU 110. The mobility of the handheld device may allow for enhanced reliability of wireless communication. In some examples, the handheld device 840 may include a charger circuit 842 for wirelessly charging a power source for powering up the motor such as located inside the IPU

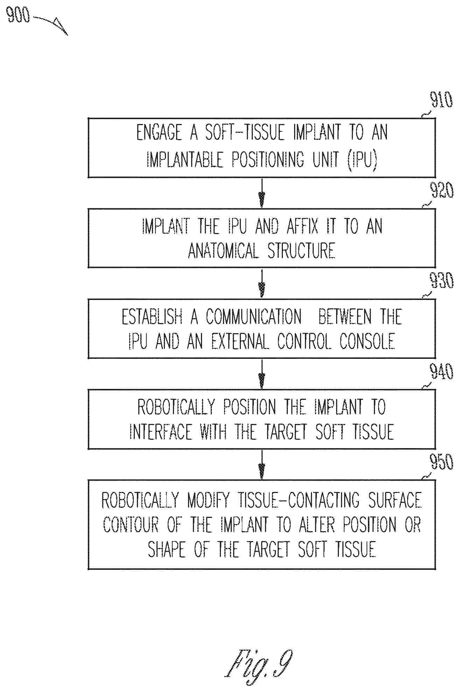

FIG. 9 illustrates, by way of example and not limitation, a method 900 for positioning a soft-tissue implant into a target implantation site via a robotically assisted and dynamically controlled tissue manipulator system, such as the robotic soft-tissue manipulator system 100. In an example, the method 900 may be used to operate the robotically controlled tissue manipulator system to position and manipulate a thyroplasty implant (such as the those illustrated in FIGS. 5-7) inside patient voice box. The thyroplasty implant may interface with a vocal cord, and alter the position or shape of the vocal cord to treat or alleviate symptoms of voice disorders such as due to vocal cord paralysis or presbylaryngis. The method 900, or a modification thereof, may alternatively be used to operate the robotically controlled tissue manipulator system to deliver, steer, position, modify, or extract other types of implants or prosthesis. Examples of such implants may include leads, catheter, guidewire, or other mechanical or electrical devices. The implants may be used for diagnosing a disease or other conditions, or alternatively or additionally be used in the cure, mitigation, treatment, or prevention of disease, such as implantable electrodes for delivering electrostimulation at cardiac, neural, muscular, or other tissues.

The method 900 commences at step 910, where the soft-tissue implant may be engaged to an implantable positioning unit (IPU), such as the IPU discussed above with reference to FIGS. 3-6. The IPU includes mechanical and electrical components for controlling the motion of the implant. The soft-tissue implant may be coupled to an elongate member detachably engaged to the IPU via a coupling unit. The coupling unit may include motorized actuation via rollers, screws, gears, or rack-pinion, among others. In an example, the coupling unit may comprise a set of rollers including a drive wheel and an idler wheel arrangement as illustrated in FIGS. 3-5. The elongate member may be fed through the IPU via an entrance port and an exit port, and compression-engaged between the driver wheel and the idler wheel. The idler wheel may be spring-biased and compress against the driver wheel, via a torsion spring. In some examples, the torsion spring may be manually biased to release the compression and open the space between the drive wheel and the idler wheel to accommodate the elongate member into the IPU.

At 920, the IPU may be implanted and affixed to an anatomical structure. In an example of robotic positioning a thyroplasty implant to adjust position and shape of a vocal cord, the IPU 110 may be anchored to patient thyroid cartilage, as illustrated in FIGS. 6A-6B. Various electrical and mechanical component of the IPU may be enclosed in a housing made of biocompatible materials, such as Silastic, goretex, biocompatible metals or polymer. The IPU may be sized and shaped to facilitate affixation. The IPU may include a fixation member, such as one or more of a screw, a pin, a nail, a wire, a hook, a barb, a helix, a suture, or a magnet. Additionally or alternatively, the fixation member may include one or more of self-drilling screws, self-tapping screws, or self-piercing screws. The IPU may have an exterior contact surface with a rough texture, or equipped with one or more gripping elements, such as spikes, pins, or barbs protruding from the exterior surface that can provide sufficient friction or gripping force to stabilize the IPU on the anchoring tissue. In some examples, the electrical and mechanical components of the IPU 110 may be packaged into separate housings that can be anchored to different anatomical structures, as illustrated in FIGS. 5A-5B.

At 930, a communication link is established between an IPU and an external control console, such as the external control console 120. The communication link may include a wired connection or a wireless connection, such as a Bluetooth protocol, a Bluetooth low energy protocol, a near-field communication (NFC) protocol, Ethernet, IEEE 802.11 wireless, an inductive telemetry link, or a radio-frequency telemetry link, among others. The external controller console may include dedicated hardware/software system that can robotically control the IPU to propel the elongate member at specific rate, direction, or distance, thereby positioning the soft-tissue implant at the target site. The external control console may additionally receive information acquired by sensors within the IPU, or measurement data from external systems that can be directly related to implant position

At 940, the soft-tissue implant may be positioned into the target site through the robotic control of the IPU. The external control console may generate an implant motion control signal according to the motion control instructions provided by the user. In response to the motion control signal, the IPU 110 may move the soft-tissue implant at specific movement rate, movement direction or orientation, or distance. The IPU 110 may additionally or alternatively control the amount of force imposed on the elongate member. In some examples, the motion control signal may control multiple motors configured to drive different modes of motion (e.g., translational or rotational motions) on the same elongate member, or to drive different elongate members, as illustrated in FIGS. 6A-6B.

Once the soft-tissue implant has been positioned at the target site and interfaces with the target soft-tissue, the soft-tissue implant may be securely attached to the target soft tissue, such as using suture holes or other active fixation mechanisms. Biocompatible material may be used at the soft tissue interface to promote tissue ingrowth and integration. In the example of vocal cord modification via a thyroplasty implant as illustrated in FIGS. 6A-6B, such a secure attachment allows the soft-tissue implant to not only push (medialize) the vocal cord to restore or improve glottic incompetence arising from vocal cord paralysis and thus to improve vocalization, but also to pull (lateralize) the vocal cord to weaken vocal cord closure or to enlarge glottis aperture to improve airway opening and ventilation. After the attachment of the implant to the target soft tissue, the implant may remain attached to the elongate member. This allows for post-surgical adjustment of the position of soft-tissue implant (e.g., revision of an existing thyroplasty implant to adjust vocal cord position or shape), yet without the need to reattach the soft-tissue implant to the elongate member.

At 950, the shape or physical dimension of at least a portion of the soft-tissue implant may be controllably adjusted to alter the position or shape of at least a portion of the soft tissue. The soft-tissue implant may include an array of micro-actuators configured to change the contour of the implant surface that interfaces with the soft-tissue, as illustrated in FIG. 7A. The micro-actuators may be based on voltage-controlled piezoelectric materials. In response to an implant contour control signal received from the external control console, the IPU may activate the micro-actuators can alter the tissue-contacting surface contour, thereby causing changes in shape or position of the target soft tissue.

The method 900 discussed herein may be used for initial implantation of the IPU for soft-tissue implant deployment and positioning. The method 900 may be modified for use in a revision procedure to modify an existing soft-tissue implant. As the IPU may have been implanted in the initial implantation and coupled to the soft-tissue implant, the method 800 may instead begin at 930 to establish a communication between an external control console and the implanted IPU, and robotically move the implant or alter the tissue-contacting surface contour to re-optimize the position and conformation of the target soft tissue, such as medializing or lateraling a vocal cord to improve vocalization. A wireless communication between the external control console and the previously implanted IPU allows a surgeon to perform non-invasive, transcutaneous control of implant position and conformation to optimize patient vocal quality as age and other factors cause the laryngeal anatomy to evolve over time.

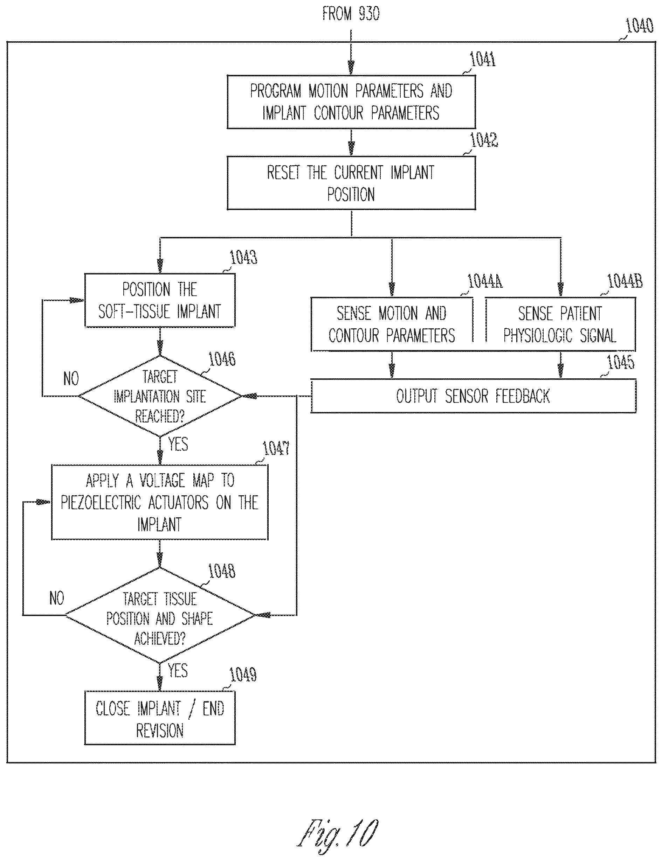

FIG. 10 illustrates, by way of example and not limitation, a method 1040 for robotically controlled positing and manipulation of a soft-tissue implant. The method 1040 is an embodiment of the steps 940 and 950 of the method 900 as illustrated in FIG. 9. In an example, the method 1040 may be used to operate the robotically controlled tissue manipulator system to position and manipulate a thyroplasty implant based on at least sensor feedback on the position of the implant, motion the implant, or the force or friction applied to the implant, and/or other patient physiologic responses such as respiration or muscle electrical signal.

Once the IPU is implanted and fixed and the communication established between the IPU and the eternal control console, a user may program one or more motion control parameters at 1041, such as via the user interface module 121 of the external control console 120. The motion control parameters may characterize desired motion of the elongate member of the implant. Examples of the motion parameters may include a target movement rate, a target movement direction or orientation, a target movement distance, a target position of a distal end of the elongate member, or a target amount of force imposed on the elongate member. In addition to the motion control parameters, a user may program one or more implant contour control parameters at 1041. The implant contour control parameters may include desired contour or topography of the tissue-contacting surface of the soft-tissue implant or a voltage map specifying voltages to be applied to respective piezoelectric actuators to produce the desired contour or topography of the implant surface. In some examples, a predetermined implant delivery protocol may be programmed into the system. The implant delivery protocol defines target values of a plurality of motion parameters. A user may adjust one or more motion control parameters or contour control parameters, modify an existing implant delivery protocol, or switch to a different implant delivery protocol during the implant delivery procedure.

Following the programming of motion and contour control parameters, the soft-tissue implant may be robotically advanced via the control console or one or more of the peripheral input controls coupled to the control console. At 1042, the current implant position may be reset to zero, such as by a short press of the foot pedal. At 1043, the implant may be positioned to the target site in accordance with the programmed motion control parameters. The motion of the implant may be activated by a surgeon using the control buttons on the control console, or a peripheral control device, such as a foot pedal or a handheld device. The movement of the implant may be activated at intervals of a predetermined step size. In an example of implantation of a thyroplasty implant, the target movement distance may range from 0.1 - 20 millimeter (mm). The target movement rate is approximately at 100-micron intervals.

During positioning and manipulation of the soft-tissue implant, one or more sensors may sense information about position and motion of the implant at 1044A. The sensor may be positioned at the motor, the power transmission unit, or inside the IPU such as at the drive wheel or idler wheel. In addition to impla nt position and motion information other sensor information about the shape, contour, or topography of the tissue-contacting surface of the soft-tissue implant, such as before and after applying voltage to the micro-actuators, may also be acquired.

Additionally or alternatively, patient physiologic signals may be sensed at 1044B during the implant positioning and manipulation. In an example of implantation or revision of a thyroplasty implant, intraoperative patient voice feedback may be acquired using a microphone coupled to the interface module 121. Voice quality may be analyzed to provide an indication of improvement in voice quality.

At 1045, the sensor feedback on implant position, motion, shape and contour, and the patient physiologic signal may be transmitted to the control console and output to a user or a process. In an example, a human-perceptible presentation of the sensed feedback, including one or more parameters on the position of the implant, motion of the implant, or the force or friction applied to the implant motion, may be generated. The presentaton may include real-time visual or audible notification with specified patterns corresponding to different types of events encountered during implantation. The audible and visual feedback may also signal to the user that the sensed implant position, motion, or the forces has exceed the target parameter values such as programmed by the user.

The sensed implant information and patient physiologic signal may be used as feedback to generate the motion control signal to adjust the position and motion of the soft-tissue implant. At 1046, the sensor feedback and/or patient physiologic response may be checked to determine whether target site has been reached. In an example, a target site is reached if the sensed distance of insertion reaches the user programmed target distance within a specified margin. A visual indicator, such as a light emitting diode (LED) or an on-screen visual indicator on the display screen with specified color or pattern may signal to the user a successful positioning of the implant at the target site. Alternatively or additionally, an audial notification, such as a beep or an alarm with a specific tone, frequency, or a specific pattern (e.g., continuous, intermittent, pulsed, sweep-up, or sweep-down sound) may go off to signal to the user successful positioning of the implant at the target site.

If the target site is not reached, then the delivery and positioning process may be continued at 1046. If at 1046 it is determined that the target site has been reached, then at 1047, a voltage map may be applied to piezoelectric actuators on the implant. The micro-actuators may be based on voltage-controlled piezoelectric materials, such that the physical dimensions of the piezoelectric actuator array may change in proportion to the applied voltage. The voltage map specifies voltages respectively applied to the piezoelectric actuators to change tissue-contacting surface contour, thereby causing changes in shape or position of the target soft tissue. The soft-tissue implant is capable of conformational changes in multiple axis and degrees of freedom (anterior, posterior, medial, lateral), as illustrated in FIGS. 7B-7C.

The sensed implant contour parameters at 1044A and the sensed patient physiologic signal at 1044B may be used as feedback to generate an implant contour control signal to adjust the contour and topography of the tissue-contacting surface of the soft-tissue implant, thereby modifying the position and shape of the target soft-tissue. At 1048, the sensor feedback and/or patient physiologic response may be checked to determine whether the target soft tissue has reached a desired position and shape. In an example of thyroplasty for vocal cord adjustment, a desired position and shape is reached if patient intraoperative vocalization attains a specific quality. If no ideal tissue position or shape is reached, then the voltage map may be adjusted to continue modifying the shape of the contacting surface of the implant at 1047. If at 1048 it is determined that ideal tissue position or shape is reached, then at 1049, the implant positioning and modification process terminates. For an initial implantation, the surgical opening for IPU implantation can be closed. If it is a post-surgical implant revision procedure, the communication between the IPU and the external control console may be disconnected.

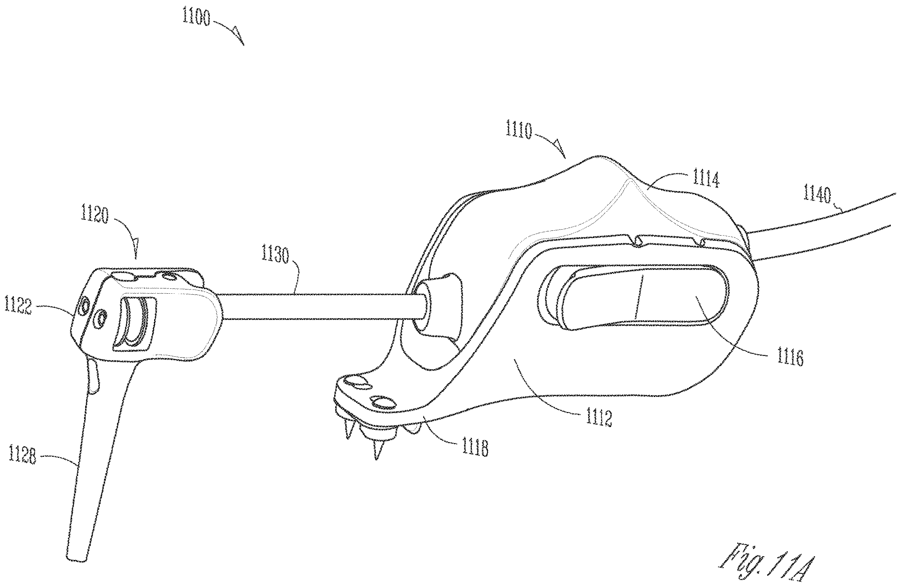

FIGS. 11A-11D illustrate, by way of example and not limitation, diagrams of different views of an implantable positioning unit (IPU) 1100 for engaging an elongate member, such as the elongate member 141 or the elongate member 301. The IPU 1100 is a variant of the IPU 300B, which can be used to robotically deliver and position an implant attached to the elongate member into a target site or to manipulate soft tissue, such as a cochlear or a vocal cord.

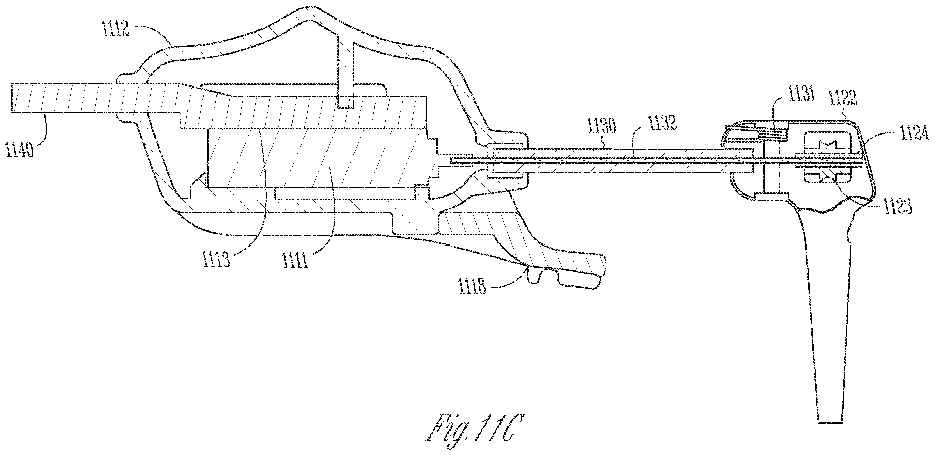

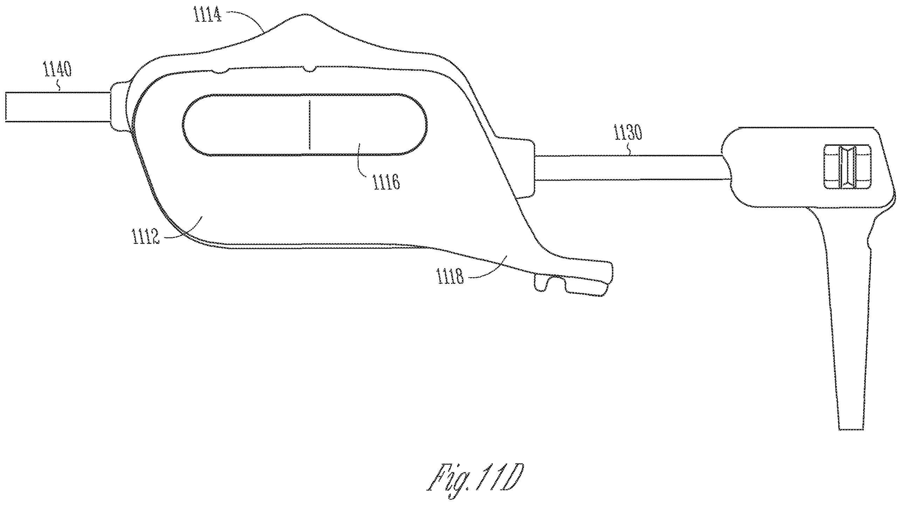

FIG. 11A illustrates a three-dimensional external view of the IPU 1100. FIGS. 11C and 11D illustrate respectively a cross-section view and a sideview of the IPU 1 100. Similar to the IPU 300B, the IPU 1100 includes a power system contained in a separate housing than a coupling unit (comprising a drive wheel and an idle wheel) for engaging the elongate member. The power system comprises a motor and motor control circuitry that provide driving force to the coupling unit. As illustrated in FIG. 11A, the IPU 1100 includes a slidable control box 1110 and an implant drive head 1120. The slidable control box 1110 includes a case 1112, a sliding member 1114, a case lock 1116, and a base mount 1118. The sliding member 1114 allows for user gripping and sliding the slidable control box 1110 linearly for optimal placement of the slidable control box 1110 on an anatomical surface (e.g., patient skull). The case lock 1116, which can be a toggle switch located on both sides of the case 1112, can lock or unlock the slidable control box 1110 at a position when pressed The base mount 1118 can be sized and shaped to conform to the anatomical surface, and can include anchoring members (e.g., self-tapping, captive bone screw holes) that secure the slidable control box 1110 thereon. In some examples, the base mount 1118 can be C-shaped to hold and compress the sliding case 1112 therein. This allows linear movement and increased travel range of the slidable control box 1110 and drive head 1120 for optimal positioning of the drive head 1120 and varying anatomical sizes.

As illustrated in FIG. 11C, enclosed within the case 1112 includes an electric motor 1111 that can generate driving force and motion, and a motor controller 1113 (e.g., a printed circuit board) that can generate motion control signal to control movement of the electric motor 1111. The electric motor 1111 and the motor controller 1113 may be respectively connected to a power source and an external control computer via a power/communication cord 1140, such as a USB cable.

The implant drive head 1120 can be connected to the slidable control box 1110 via an adjustable arm 1130, such as an adjustable Gooseneck arm. The adjustable arm 1130 can be a flexible, semirigid arm that allows for multiple depth-of-field (DOF) adjustment of the drive head 1120 at multiple, different angles to the insertion implant site, providing adjustable stability of the drive head 1120. In an example, the adjustable arm 1130 can be bended to adjust the position of the implant drive head 1120. This allows for easy advancement of the elongate member and the associated implant into the target site (e.g., cochlea or vocal cord).

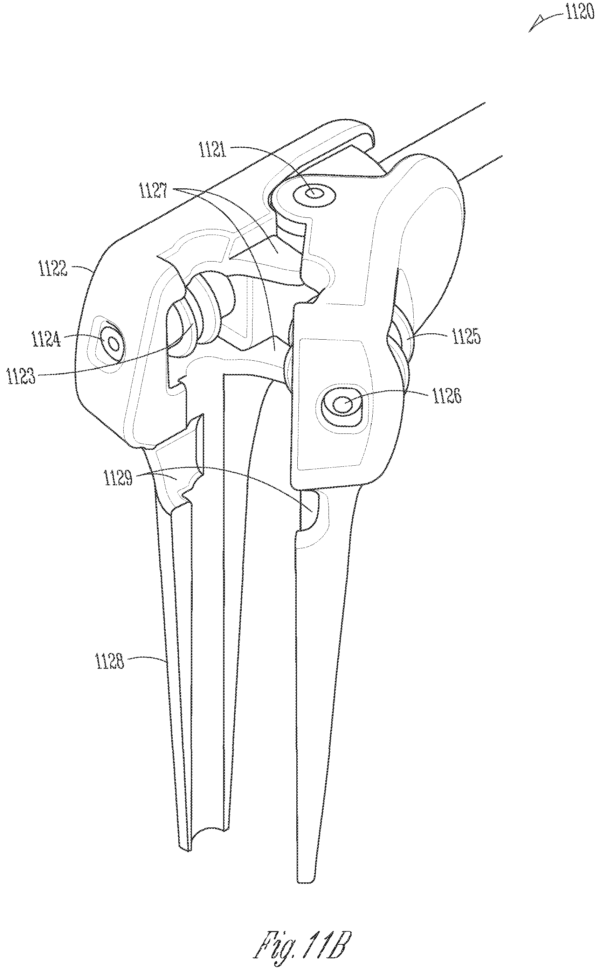

The implant drive head 1120 includes a drive housing 1122 that can house drive mechanism, an introducer sheath, and sheath components. FIG. 11B illustrates a cutaway view of the implant drive head 1120. The drive housing 1122 comprises two symmetrical housing halves interconnected via a hinge 1121. The hinge 1121 allows for opening and closing of the drive housing 1122 to engage and disengage with the elongated member or electrode of varying sizes, geometry, and diameter. In some examples, one or more backstops 1127 can be included inside the drive housing 1122 to prevent the elongate member or the electrode therewith from reaching the hinge 1121 during engagement, thereby prevening inadvertent damage to the elongate member or the electrode therewith.

The drive mechanism inside the drive housing 1122 includes a drive wheel 1123 and an idle wheel 1125, and can include a torsion spring 1131. As similarly discussed above such as with reference to FIGS. 3A-3B, the drive wheel 1123 rotates to insert or retract elongate member through sheath, and the idle wheel 1125 rotates to keep the elongate member aligned with the drive wheel 1123. A drive pin 1124 may be included to improve smooth rotation of the drive wheel 1123, and an idle pin 1126 may be included to improve smooth rotation of the idle wheel 1125. As illustrated in FIG. 11C, torque may be transmitted from the electric motor 1111 to the drive wheel 1123 via a torque cable 1132, at least a portion of which can be enclosed in the adjustable arm 1130. The torsion spring 1131 allows the drive head 1120 to open and close, and provides compression on the elongate member for frictional motion.

The implant drive head 1120 includes a sheath 1128 that provides lateral and peripheral support to move the flexible elongate member inside the sheath 1128. In an example, a loading access 1129, such as a slit or notch, can be included in the sheath 1128 for accessing and loading the elongated member into the drive head 1120 after the housing halves are closed. A distal end of the sheath 1128 opens to form a guide track slot, which can be a specially shaped tip to control final orientation of the implant placement based on the implant geometry.

Various embodiments are illustrated in the figures above. One or more features from one or more of these embodiments may be combined to form other embodiments.

The method examples described herein can be machine or computer-implemented at least in part. Some examples may include a computer-readable medium or machine-readable medium encoded with instructions operable to configure an electronic device or system to perform methods as described in the above examples. An implementation of such methods may include code, such as microcode, assembly language code, a higher-level language code, or the like. Such code may include computer readable instructions for performing various methods. The code can form portions of computer program products. Further, the code can be tangibly stored on one or more volatile or non-volatile computer-readable media during execution or at other times.

The above detailed description is intended to be illustrative, and not restrictive. The scope of the disclosure should be determined with reference to the appended claims.

## Claims

1. A system (100) for robotically assisted manipulation of an implant (140), the system comprising:
a drive head (1120) configured to engage an elongate member (141; 301) of the implant and robotically deliver and position the implant into a target implantation site, the drive head including:
a housing (1122) including a first half and a second half, the first half coupled to the second half with a hinge (1121), to enable the housing to receive the elongate member;
a sheath (1128) including separable first and second portions associated respectively with the first and second halves of the housing (1122), the separable first and second portions of the sheath forming a guide track configured to accommodate and channel the elongate member (141; 301) in a linear motion; and
a coupling unit including a drive wheel (1123) and an idle wheel (1125) disposed respectively within the first and second halves of the housing (1122), the coupling unit configured to translate the elongate member (141; 301) using the wheel within the guide track, in response to a motion control signal;
a power system coupled to the coupling unit, the power system including a motor (1111) and motor control circuitry (1113) positioned within a case (1112) and configured to drive the drive wheel (1123) of the coupling unit; and
a control console (120; 820) communicatively coupled to the power system, the control console including a controller circuit (822) configured to generate the motion control signal, to robotically deliver and position the implant into the target implantation site,
wherein the guide track includes a first longitudinal track portion and a second longitudinal track portion, the first longitudinal track portion defined by the first half of the housing and the second longitudinal track portion defined by the second half of the housing.

2. The system of claim 1, wherein the drive head (1120) includes:
an entrance port to receive the elongate member; and
an exit port to release the elongate member from the drive head (1120), wherein the guide track is located between the entrance port and the exit port.

3. The system of any of claims 1-2, wherein the drive head (1120) includes an implant access port (1129) open to the first and the second longitudinal track portions, the implant access port configured to enable access to the elongate member (141) from a location external to the drive head.

4. The system of any of claims 1-3, wherein the first and the second longitudinal track portions are in parallel and configured to accommodate the elongate member (141) and to limit rotation or twisting during translation of the elongate member.

5. The system of any of claims 1-4, wherein the first and the second longitudinal track portions define a groove having a distal tip shaped to control placement orientation of the implant based on a geometry of the implant.

6. The system of any of claims 1-5, comprising the implant (140), wherein a first portion of the implant includes the elongate member (141) and a second portion of the implant includes an electrode array, the electrode array detachably connected to the elongate member.

7. The system of any one of claims 1 to 6 further comprising: a user interface (121) configured to display a graphical user interface to a user, the user interface in communication with the control console (120), the user interface configured to receive an input from a user to operate the control console (120) and the drive head (1120) using the user interface.

8. The system of claim 7, wherein the user interface (121) includes a touchscreen and the graphical user interface is displayed on the touchscreen, the touchscreen configured to receive contact input from the user to operate the control console (120) and the drive head (1120).

9. The system of any of claims 7-8, wherein the first and the second longitudinal track portions include a groove configured to accommodate the elongate member (141; 301) and limit rotation or twisting during translation of the elongate member.

10. The system of any of claims 7-9, wherein the sheath at least partially encloses the elongate member (141; 301), and wherein the coupling unit is configured to engage and move the elongate member (141; 301) out of the drive head (1120) through the sheath (1128).

## Patentansprüche

1. Ein System (100) zur robotergestützten Manipulation eines Implantats (140), wobei das System Folgendes beinhaltet:
einen Antriebskopf (1120), der dazu ausgelegt ist, Eingriff mit einem länglichen Element (141; 301) des Implantats zu nehmen und das Implantat robotisch an einen Zielimplantationsort zu bringen und darin zu positionieren, wobei der Antriebskopf Folgendes umfasst:
ein Gehäuse (1122), das eine erste Hälfte und eine zweite Hälfte umfasst, wobei die erste Hälfte mit einem Scharnier (1121) mit der zweiten Hälfte gekoppelt ist, um es dem Gehäuse zu ermöglichen, das längliche Element aufzunehmen;
eine Hülse (1128), die einen ersten und zweiten voneinander trennbaren Abschnitt umfasst, die mit der ersten bzw. zweiten Hälfte des Gehäuses (1122) assoziiert sind, wobei der erste und zweite voneinander trennbare Abschnitt der Hülse eine Führungsbahn bilden, die dazu ausgelegt ist, das längliche Element (141; 301) unterzubringen und in einer linearen Bewegung zu lenken; und
eine Koppeleinheit, die ein Triebrad (1123) und ein Zwischenrad (1125) umfasst, die innerhalb der ersten bzw. zweiten Hälfte des Gehäuses (1122) angeordnet sind, wobei die Koppeleinheit dazu ausgelegt ist, das längliche Element (141; 301) unter Verwendung des Rads innerhalb der Führungsbahn als Reaktion auf ein Bewegungssteuersignal zu verschieben;
ein Stromversorgungssystem, das mit der Koppeleinheit gekoppelt ist, wobei das Stromversorgungssystem einen Motor (1111) und Motorsteuerschaltungen (1113) umfasst, die in einer Ummantelung (1112) positioniert sind und dazu ausgelegt sind, das Triebrad (1123) der Koppeleinheit anzutreiben; und
eine Steuerkonsole (120; 820), die kommunikativ mit dem Stromversorgungssystem gekoppelt ist, wobei die Steuerkonsole eine Steuergerätschaltung (822) umfasst, die dazu ausgelegt ist, das Bewegungssteuersignal zu erzeugen, um das Implantat robotisch an den Zielimplantationsort zu bringen und darin zu positionieren,
wobei die Führungsbahn einen ersten längs verlaufenden Bahnabschnitt und einen zweiten längs verlaufenden Bahnabschnitt umfasst, wobei der erste längs verlaufende Bahnabschnitt durch die erste Hälfte des Gehäuses definiert ist und der zweite längs verlaufende Bahnabschnitt durch die zweite Hälfte des Gehäuses definiert ist.

2. System gemäß Anspruch 1, wobei der Antriebskopf (1120) Folgendes umfasst:
eine Eintrittsöffnung, um das längliche Element aufzunehmen; und
eine Austrittsöffnung, um das längliche Element aus dem Antriebskopf (1120) freizugeben, wobei sich die Führungsbahn zwischen der Eintrittsöffnung und der Austrittsöffnung befindet.

3. System gemäß einem der Ansprüche 1-2, wobei der Antriebskopf (1120) eine zu dem ersten und dem zweiten längs verlaufenden Bahnabschnitt hin offene Implantatzugangsöffnung (1129) umfasst, wobei die Implantatzugangsöffnung dazu ausgelegt ist, den Zugang zu dem länglichen Element (141) von einer außerhalb des Antriebskopfs liegenden Stelle zu ermöglichen.

4. System gemäß einem der Ansprüche 1-3, wobei der erste und der zweite längs verlaufende Bahnabschnitt parallel zueinander liegen und dazu ausgelegt sind, das längliche Element (141) unterzubringen und die Rotation oder Verdrehung während der Verschiebung des länglichen Elements einzuschränken.

5. System gemäß einem der Ansprüche 1-4, wobei der erste und der zweite längs verlaufende Bahnabschnitt eine Auskehlung mit einer distalen Spitze definieren, die so gestaltet ist, dass sie die Platzierungsausrichtung des Implantats basierend auf einer Geometrie des Implantats steuert.

6. System gemäß einem der Ansprüche 1-5, das das Implantat (140) beinhaltet, wobei
ein erster Abschnitt des Implantats das längliche Element (141) umfasst und ein zweiter Abschnitt des Implantats eine Elektrodenanordnung umfasst, wobei die Elektrodenanordnung abnehmbar mit dem länglichen Element verbunden ist.

7. System gemäß einem der Ansprüche 1 bis 6, das ferner Folgendes beinhaltet:
eine Benutzerschnittstelle (121), die dazu ausgelegt ist, einem Benutzer eine grafische Benutzerschnittstelle anzuzeigen, wobei die Benutzerschnittstelle in Kommunikation mit der Steuerkonsole (120) ist, wobei die Benutzerschnittstelle dazu ausgelegt ist, eine Eingabe von einem Benutzer zu empfangen, um die Steuerkonsole (120) und den Antriebskopf (1120) unter Verwendung der Benutzerschnittstelle zu bedienen.

8. System gemäß Anspruch 7, wobei die Benutzerschnittstelle (121) einen Touchscreen umfasst und die grafische Benutzerschnittstelle auf dem Touchscreen angezeigt wird, wobei der Touchscreen dazu ausgelegt ist, Kontakteingaben von dem Benutzer zu empfangen, um die Steuerkonsole (120) und den Antriebskopf (1120) zu bedienen.

9. System gemäß einem der Ansprüche 7-8, wobei der erste und der zweite längs verlaufende Bahnabschnitt eine Auskehlung umfassen, die dazu ausgelegt ist, das längliche Element (141; 301) unterzubringen und die Rotation oder Verdrehung während der Verschiebung des länglichen Elements einzuschränken.

10. System gemäß einem der Ansprüche 7-9, wobei die Hülse das längliche Element (141; 301) mindestens teilweise umschließt und wobei die Koppeleinheit dazu ausgelegt ist, das längliche Element (141; 301) zu erfassen und es aus dem Antriebskopf (1120) heraus durch die Hülse (1128) zu bewegen.

## Revendications

1. Un système (100) destiné à la manipulation assistée par robot d'un implant (140), le système comprenant :
une tête d'entraînement (1120) configurée pour se mettre en prise avec un élément allongé (141 ; 301) de l'implant et poser et positionner par robot l'implant jusque dans un site cible d'implantation, la tête d'entraînement incluant :
un boîtier (1122) incluant une première moitié et une deuxième moitié, la première moitié étant accouplée à la deuxième moitié avec une charnière (1121), afin de permettre au boîtier de recevoir l'élément allongé ;
une gaine (1128) incluant des première et deuxième portions séparables associées respectivement aux première et deuxième moitiés du boîtier (1122),
les première et deuxième portions séparables de la gaine formant une voie de guidage configurée pour accueillir et diriger l'élément allongé (141 ; 301) dans un mouvement linéaire ; et
une unité d'accouplement incluant une roue motrice (1123) et une roue folle (1125) disposées respectivement au sein des première et deuxième moitiés du boîtier (1122), l'unité d'accouplement étant configurée pour translater l'élément allongé (141 ; 301) à l'aide de la roue au sein de la voie de guidage, en réponse à un signal de commande de mouvement ;
un système d'alimentation accouplé à l'unité d'accouplement, le système d'alimentation incluant un moteur (1111) et des circuits de commande de moteur (1113) positionnés au sein d'un carter (1112) et configurés pour entraîner la roue motrice (1123) de l'unité d'accouplement ; et
une console de commande (120 ; 820) accouplée en communication au système d'alimentation, la console de commande incluant un circuit de commande (822) configuré pour générer le signal de commande de mouvement, afin de poser et de positionner par robot l'implant jusque dans le site cible d'implantation,
dans lequel la voie de guidage inclut une première portion de voie longitudinale et une deuxième portion de voie longitudinale, la première portion de voie longitudinale étant définie par la première moitié du boîtier et la deuxième portion de voie longitudinale étant définie par la deuxième moitié du boîtier.

2. Le système de la revendication 1, dans lequel la tête d'entraînement (1120) inclut :
un orifice d'entrée afin de recevoir l'élément allongé ; et
un orifice de sortie afin de libérer l'élément allongé de la tête d'entraînement (1120), dans lequel la voie de guidage est située entre l'orifice d'entrée et l'orifice de sortie.

3. Le système de n'importe lesquelles des revendications 1 à 2, dans lequel la tête d'entraînement (1120) inclut un orifice d'accès à l'implant (1129) ouvert sur la première et la deuxième portion de voie longitudinale, l'orifice d'accès à l'implant étant configuré pour permettre l'accès à l'élément allongé (141) depuis un emplacement externe à la tête d'entraînement.

4. Le système de n'importe lesquelles des revendications 1 à 3, dans lequel la première et la deuxième portion de voie longitudinale sont parallèles et configurées pour accueillir l'élément allongé (141) et pour limiter une rotation ou une torsion durant la translation de l'élément allongé.

5. Le système de n'importe lesquelles des revendications 1 à 4, dans lequel la première et la deuxième portion de voie longitudinale définissent une rainure ayant une extrémité distale façonnée afin de commander une orientation de mise en place de l'implant sur la base d'une géométrie de l'implant.

6. Le système de n'importe lesquelles des revendications 1 à 5, comprenant l'implant (140), dans lequel une première portion de l'implant inclut l'élément allongé (141) et une deuxième portion de l'implant inclut un réseau d'électrodes, le réseau d'électrodes étant connecté de façon amovible à l'élément allongé.

7. Le système de n'importe laquelle des revendications 1 à 6 comprenant en outre :
une interface utilisateur (121) configurée pour afficher une interface utilisateur graphique pour un utilisateur, l'interface utilisateur étant en communication avec la console de commande (120), l'interface utilisateur étant configurée pour recevoir une saisie d'un utilisateur afin de faire fonctionner la console de commande (120) et la tête d'entraînement (1120) à l'aide de l'interface utilisateur.

8. Le système de la revendication 7, dans lequel l'interface utilisateur (121) inclut un écran tactile et l'interface utilisateur graphique est affichée sur l'écran tactile, l'écran tactile étant configuré pour recevoir une saisie de contact de l'utilisateur afin de faire fonctionner la console de commande (120) et la tête d'entraînement (1120).

9. Le système de n'importe lesquelles des revendications 7 à 8, dans lequel la première et la deuxième portion de voie longitudinale incluent une rainure configurée pour accueillir l'élément allongé (141 ; 301) et limiter une rotation ou une torsion durant la translation de l'élément allongé.

10. Le système de n'importe lesquelles des revendications 7 à 9, dans lequel la gaine entoure au moins partiellement l'élément allongé (141 ; 301), et dans lequel l'unité d'accouplement est configurée pour se mettre en prise avec et déplacer l'élément allongé (141 ; 301) hors de la tête d'entraînement (1120) à travers la gaine (1128).
